# EUROPEAN PATENT APPLICATION

(11) **EP 4 494 646 A1**
(43) Date of publication of application: **22.01.2025**
(21) Application number: 22931308.5
(22) Date of filing: 15.03.2022
(51) Int. Cl.: A61K 36/185, A61K 31/7048, A61P 25/00, A61P 13/12, A61P 9/00

(54) **EFFECTIVE PARTS OF FLAVONOIDS FROM FLOS ABELMOSCHUS MANIHOT, AND PREPARATION METHOD THEREFOR AND USE THEREOF**

(71) Applicant: Suzhong Pharmaceutical Group Co., Ltd., Jiangsu 225500 (CN); Jiangsu Suzhong Pharmaceutical Research Institute Co., Ltd., Nanjing, Jiangsu 210000 (CN)
(72) Inventor: TANG, Haitao, Taizhou, Jiangsu 225500 (CN); WANG, Dandan, Taizhou, Jiangsu 225500 (CN); GE, Haitao, Taizhou, Jiangsu 225500 (CN); WANG, Dianguang, Taizhou, Jiangsu 225500 (CN); PENG, Xiaolan, Taizhou, Jiangsu 225500 (CN); LIANG, Hui, Taizhou, Jiangsu 225500 (CN); WANG, Fujiang, Taizhou, Jiangsu 225500 (CN)
(74) Representative: Murgitroyd & Company
(86) International application number: PCT/CN2022/080818
(87) International publication number: WO 2023/173268

(57) **Abstract**

The invention relates to a flavonoid active fraction of an Abelmoschi corolla, a preparation method and an application thereof, and belongs to the field of traditional Chinese pharmaceutics. A high-purity Abelmoschi corolla flavonoid active fraction is prepared, which is found to have better therapeutic activity for kidney disease, and especially have a significant therapeutic effect for diabetic kidney disease and lupus nephritis, or contrast-induced kidney injury and eye diseases. Meanwhile, the Abelmoschi corolla flavonoid active fraction described in the invention also has good safety.

## Description

### TECHNICAL FIELD

The present invention belongs to the field of traditional Chinese pharmaceutics, and more particularly, relates to a flavonoid active fraction of an Abelmoschi corolla and an application thereof.

### BACKGROUND

Medicinal Abelmoschi corolla is the dried corolla of Abelmoschus Manihot (L.) Medic in Malvaceae, which tastes sweet and is cold, and acts on kidney and bladder channels. Having the efficacy of eliminating dampness and heat, resolving toxin and dispersing swelling, the Abelmoschi corolla can be used to treat toxic swelling of carbuncle, and burns caused by a flame or hot liquid. The Abelmoschi corolla was first recorded in *Materia Medica of Jia You Reign,* and according to the records of the *Materia Medica of Jia You Reign,* the Abelmoschi corolla mainly used for treating patients with malignant sore and pus for a long time via external application of its powder. According to the records in *Compendium of Materia Medica,* the Abelmoschi corolla smells sweet, is cold and smooth and non-toxic, and as a sovereign drug for treatment of sore diseases, the Abelmoschi corolla is mainly used for treating patients with malignant sore and pus for a long time via external application of its powder.

A flavonoid compound is one of the main constituents of Abelmoschi corolla, and also one of its pharmacological active ingredients. Huangkui Capsule has been on the market for many years, and its main active ingredient is the extract of Abelmoschi corolla. Studies have confirmed that the flavonoid constituent in the Abelmoschi corolla extract not only has obvious therapeutic effect on sores, but also has significant protective effect on heart, brain and tissue damage caused by ischemia. Additionally, numerous literature reports have shown that a total flavonoid in the Abelmoschi corolla may have good effects in terms of resistance against inflammation, relieving of fever and pain, protection against ischemic damage to the heart and brain, reduction of blood sugar level and resistance against virus. For example, the patent CN201210082553.7 discloses a total flavonoid extract of the Abelmoschi corolla comprising a flavonoid in the weight ratio of (1 1 to 16): (2.5 to 6): (4 to 6.5) of gossypetin-3'-glucoside, quercetin-3'-glucoside and isoquercitrin, which can be used in preparation of a drug for treating nephritis diseases. And the Patent CN200610097615.6 discloses a total flavonoid extract of the Abelmoschi corolla which comprises 50% to 90% of total flavonoid by weight. This total flavonoid comprises 1.0% to 5.0% of quercetin-3-robinobioside, 8% to 24.0% of hyperoside, 7.0% to 20.0% of isoquercitrin, 5.0% to 15.0% of quercetin-3'-glucoside, 3.0% to 10.0% of gossypetin-3'-glucoside, 0.5% to 5.0% of myricetin, 0.5% to 5.0% of gossypetin, 2.0% to 8.0% of isoquercitrin, and several other flavonoid constituents. The total flavonoid extract of the Abelmoschi corolla can be used in preparation of a drug for treating nephritis, with a clearly qualitative and quantitative flavonoid constituent and a reliable therapeutic efficacy.

Although bioflavonoids have various therapeutic effects, the types of flavonoids are various. The bioflavonoid can now be divided into seven different subtypes, and different flavonoid constituents can produce different therapeutic effects. For example, while the bioflavonoid has been reported in the literature to be effective in preventing or treating diabetic retinopathy, compounds having five hydroxyl groups such as quercetin have a negative effect on ocular blood flow, whereas significant improvements in ocular blood flow can be obtained when the flavonoid is dehydrogenated to a flavanone. In addition, the compound that increases blood flow also leads to a significant increase in the recovery of retinal function after ischemic injury. How to develop such compounds for effective use in the treatment of eye diseases such as macular degeneration, visual fatigue and cataracts or even to enhance the effect on diabetic retinopathy is an important task for research.

Choroidal neovascularization (CNV) is found in many fundus oculi diseases, such as age-related macular degeneration (AMD), central exudative chorioretinitis and macular degeneration caused by high myopia, among which CNV secondary to AMD is the most common one, and has become one of the main causes of irreversible visual impairment in the elderly. Insufficiency of blood flow is also an influential factor in visual fatigue, and enhancing blood flow to the eyes or acting on the smooth muscle of the eyes is helpful to restore visual fatigue.

Glucose reabsorption in proximal renal tubules is mediated by sodium-glucose cotransporter (SGLT)1 and SGLT2 transporter, of which about 90% of glucose reabsorption is mediated by SGLT2 and the remaining 10% by SGLT1. An SGLT2 inhibitor can inhibit the reabsorption of glucose in renal tubules by selectively binding to SGLT2 receptors, thereby reducing the renal glucose threshold, increasing urine glucose excretion and achieving the goal of significantly reducing blood sugar level. In recent years, clinical studies have found that the SGLT2 inhibitor has renal protective effects on non-diabetic chronic kidney disease (CKD). The SGLT2 inhibitor can reduce the expression of collagen and fibronectin by reducing TGFβ-1, PAI1, STAT 1, MMP 7 and by inhibiting AGE-RAGE axis, thereby decreasing the accumulation of extracellular matrix and alleviating the progression of DN in renal fibrosis. The SGLT2 inhibitor, in addition to modulating tubuloglomerular feedback, slowing down the progression of proteinuria, and resisting against inflammation and fibrosis, can also reduce the serum uric acid (SUA) in diabetic patients. For example, compared with the control group, the SGLT2 inhibitor (Empagliflozin, Canagliflozin, Dapagliflozin, Tofogliflozin, Luseogliflozin and Ipragliflozin) significantly reduces the serum uric acid level. The SGLT2 inhibitor may be beneficial to the prevention and treatment of cardiac failure by reducing plasma volume, decreasing anterior and posterior load, and improving myocardial energy metabolism and myocardial remodeling.

Abelmoschi corolla extraction methods reported in literatures comprise ethanol reflux extraction, ultrasonic extraction and room-temperature soaking extraction. However, the Abelmoschi corolla extract has complex ingredients, a great change in contents of effective ingredients, a low extraction transfer rate, high extraction energy consumption and a large solvent dosage. Therefore, it is necessary to explore an Abelmoschi corolla drug for combined treatment of kidney diseases, especially diabetic kidney disease, eye diseases and other diseases, and an extraction process thereof.

The statistics of cases of adverse reactions of the Huangkui Capsule made by the applicant show that 194 cases of adverse reactions of the Huangkui Capsule were received in a recent year, and the statistics of manifestations of adverse drug reactions show that nausea, itching, rash, vomiting, diarrhea, and epigastric fullness and discomfort occurred more than 10 times, all of which were known adverse reactions. New ADR manifestations appearing recently comprise dizziness, headache and loss of appetite. In addition, in serious adverse reactions received, one case involved liver cell damage, which could be a signal of liver cell damage caused by the Huangkui Capsule. Therefore, it is of clinical significance to develop a new Abelmoschi corolla flavone extract to reduce side effects and to use as a drug for treating diseases.

### SUMMARY

In view of the above shortcoming, the present invention provides a flavonoid active fraction of an Abelmoschi corolla and an application thereof. According to the present invention, a high-purity Abelmoschi corolla flavonoid active fraction is prepared, which is found to have better therapeutic activity for kidney disease, such as diabetic kidney disease, contrast-induced kidney injury or lupus nephritis, and also have a therapeutic effect on eye diseases. Meanwhile, the active fraction, extract or composition of the flavonoids from the Abelmoschi corolla according to the present invention has lower side effects, and provides a better therapeutic drug for patients.

In order to solve the above technical problem, the following technical solution is used in the present invention.

In a first aspect, the present invention provides a flavonoid active fraction of an Abelmoschi corolla, comprising the following flavonoid constituents in mass ratio: a mass ratio of gossypetin-8-O-β-D-glucuronide: hyperoside: isoquercitrin: myricetin: quercetin-3'-O-glucoside being 10: 3.0 to 20: 4.0 to 18: 1.9 to 6.0: 6.0 to 20.

Specifically, the mass ratio of the gossypetin-8-O-β-D-glucuronide: the hyperoside: the isoquercitrin: the myricetin: the quercetin-3'-O-glucoside is 10: 4.0 to 17: 4.50 to 16: 1.9 to 5.5: 7.0 to 16, preferably 10: 6.0 to 15: 5.0 to 13: 2.0 to 5.0: 8.0 to 14, and further, the active fraction comprises quercetin, wherein a mass ratio of the gossypetin-8-O-β-D-glucuronide: the quercetin is 10: 1.0 to 10.0, preferably 10: 1.0 to 6.0, preferably 10: 1.5 to 5.0, still further, the active fraction comprises rutin, wherein a mass ratio of the gossypetin-8-O-β-D-glucuronide: the rutin is 10: 0.05 to 0.6, preferably 10: 0.1 to 0.4, and further preferably 10: 0.1to 0.3, and yet still further, the active fraction comprises quercetin-3-O-robinobioside, wherein a mass ratio of the gossypetin-8-O-β-D-glucuronide: the quercetin-3-O-robinobioside is 10: 0.1 to 2.5, 10: 0.1 to 0.9, preferably 10: 0.15 to 0.5.

In a second aspect, the present invention provides a flavonoid active fraction of an Abelmoschi corolla, comprising the following flavonoid constituents in mass ratio: a mass ratio of isoquercitrin: quercetin: quercetin-3'-O-glucoside: myricetin: gossypetin-8-O-β-D-glucuronide: hyperoside being 0.8 to 1.2: 0.05 to 1.6: 0.2 to 4.6: 0.05 to 1.2: 0.2 to 3.5: 0.25 to 3.6.

Specifically, the flavonoid active fraction of an Abelmoschi corolla comprises the following flavonoid constituents in mass ratio: a mass ratio of isoquercitrin: quercetin: quercetin-3'-O-glucoside: myricetin: gossypetin-8-O-β-D-glucuronide: hyperoside being 0.8 to 1.2: 0.1 to 1.2: 0.4 to 3.1: 0.10 to 0.9: 0.4 to 3.1: 0.5 to 3.0, further 0.8 to 1.2: 0.16 to 1.0: 0.6 to 2.4: 0.14 to 0.75: 0.6 to 2.5: 0.6 to 2.4, further 0.8 to 1.2: 0.2 to 0.8: 0.8 to 2.1: 0.18 to 0.6: 0.8 to 2.0: 0.7 to 2.0, further 0.8 to 1.2: 0.2 to 0.7: 0.8 to 1.6: 0.2 to 0.5: 0.8 to 1.8: 0.8 to 1.4, and further 0.8 to 1.2: 0.2 to 0.6: 1.0 to 1.2: 0.2 to 0.4: 0.9 to 1.7: 1.0 to 1.3.

Specifically, the flavonoid active fraction of an Abelmoschi corolla comprises the following flavonoid constituents in mass ratio: a mass ratio of isoquercitrin: quercetin: quercetin-3'-O-glucoside: myricetin: gossypetin-8-O-β-D-glucuronide: hyperoside being 0.8 to 1.2: 0.09 to 1.6: 0.2 to 2.0: 0.05 to 1.2: 0.5 to 3.0: 0.25 to 3.6.

Specifically, the flavonoid active fraction of an Abelmoschi corolla comprises the following flavonoid constituents in mass ratio: a mass ratio of isoquercitrin: quercetin: quercetin-3'-O-glucoside: myricetin: gossypetin-8-O-β-D-glucuronide: hyperoside being 0.8 to 1.2: 0.12 to 0.7: 0.8 to 1.6: 0.2 to 0.5: 0.85 to 1.7: 1.0 to 1.4.

More specifically, the mass ratio of isoquercitrin is 1.2 or 1.0.

More specifically, the flavonoid active fraction of an Abelmoschi corolla further comprises rutin, and a mass ratio of the isoquercitrin: the rutin is 1: 0.001 to 0.08, preferably 1: 0.005 to 0.06, preferably 1: 0.006 to 0.05, preferably 1: 0.007 to 0.04, and preferably 1: 0.009 to 0.04, or preferably 1: 0.008 to 0.03, preferably 1: 0.009 to 0.03, and further, the flavonoid active fraction comprises quercetin-3-O-robinobioside, and a mass ratio of the isoquercitrin: the quercetin-3-O-robinobioside is 10: 0.1 to 2.5, 10: 0.1 to 0.9, and preferably 10: 0.15 to 0.5.

More specifically, the flavonoid active fraction of an Abelmoschi corolla comprises a total of more than 55% of quercetin, quercetin-3'-O-glucoside, myricetin, gossypetin-8-O-β-D-glucuronide, isoquercitrin and hyperoside, preferably more than 60%, preferably 65% to 85%, and preferably 69% to 82%, further, the content of the quercetin-3'-O-glucoside is 12.1% to 25%, and still further, the content of the quercetin-3'-O-glucoside is 12.6% to 23% or 13% to 20%, 13% to 20% or 14% to 25%, or further, the content of the quercetin is higher than 0.7%, further higher than 1.0%, further 1.0% to 10%, further 1.5% to 5%, or still further, the gossypetin-8-O-β-D-glucuronide is 8.5% to 30%, further 12% to 23%.

More specifically, the active fraction comprises a total of more than 55% of quercetin, quercetin-3'-O-glucoside, myricetin, gossypetin-8-O-β-D-glucuronide, isoquercitrin, rutin and hyperoside, preferably more than 60%, preferably 65% to 85% or 66% to 84%, preferably 69% to 82% or 69% to 90%, and further the content of the quercetin-3'-O-glucoside is 12.1% to 25%, and still further the content of the quercetin-3'-O-glucoside is 12.6% to 23% or 13% to 20%.

In a third aspect, the present invention provides a plant extract comprising flavonoid constituents, wherein the plant extract comprises the following constituents in mass ratio: 10% to 25% of hyperoside, 8% to 19% of isoquercitrin, 5% to 30% of gossypetin-8-O-β-D-glucuronide, and 12.1% to 25% of quercetin-3'-O-glucoside, further 12% to 23% of hyperoside, 10% to 17% of isoquercitrin, 8.5% to 25% of gossypetin-8-O-β-D-glucuronide, and 12.6% to 23% of quercetin-3'-O-glucoside, and further 13% to 22% of hyperoside, 11% to 17% of isoquercitrin, 12% to 21% of gossypetin-8-O-β-D-glucuronide, and 13% to 20% of quercetin-3'-O-glucoside.

Specifically, the extract further comprises 2% to 10% of quercetin, preferably 2.5% to 9% of quercetin, preferably 3% to 8.5% of quercetin, and further 1.5% to 5% of quercetin, still further comprises 2% to 11% of myricetin, preferably 3% to 7% of myricetin, preferably 3% to 5% of myricetin, and further comprises 0.08% to 2.5% of quercetin-3-O-robinobioside, preferably 0.1% to 1.5%, further 0.1% to 0.9%, and still further 0.1% to 0.5%, and further, the plant is an Abelmoschi corolla or an Abelmoschus esculentus flower, which may be a complete flower or a corolla, preferably the Abelmoschi corolla.

In a fourth aspect, the present invention provides an Abelmoschi corolla extract comprising flavonoid constituents, wherein the flavonoid constituent comprises the following constituents in mass ratio: 8% to 26% of hyperoside, 12.0% to 19.8% of isoquercitrin, 8.5% to 30% of gossypetin-8-O-β-D-glucuronide, 3.0% to 4.9% or 5.1% to 6.0% of myricetin, 14% to 25% of quercetin-3'-O-glucoside, and 1.6% to 4.9% of quercetin, and further 11% to 22% of hyperoside, 12.0% to 17% of isoquercitrin, 12% to 23% of gossypetin-8-O-β-D-glucuronide, 1.6% to 4.9% or 5.1% to 9.0% of myricetin, 13% to 22% of quercetin-3'-O-glucoside, and 1.4% to 8% or 1.4% to 7.8% of quercetin.

Specifically, the extract further comprises rutin at a mass content of 0.01% to 1.0%, further 0.05% to 0.8%, further 0.09% to 0.8%, and further 0.1% to 0.6%, and further comprises 0.08% to 2.5% of quercetin-3-O-robinobioside, preferably 0.1% to 1.5%, further 0.1% to 0.9%, and still further 0.1% to 0.5%.

Specifically, the flavonoid constituent in the Abelmoschi corolla extract has a mass content more than 55%, preferably more than 60%, preferably 65% to 85% or 66% to 84%, and preferably 69% to 82% or 69% to 90%.

In a fifth aspect, the present invention provides a composition, comprising the following flavonoid constituents in mass ratio, wherein a mass ratio of isoquercitrin: quercetin: quercetin-3'-O-glucoside: myricetin: gossypetin-8-O-β-D-glucuronide: hyperoside is 1: 0.05 to 1.6: 0.2 to 4.6: 0.05 to 1.2: 0.2 to 3.5: 0.25 to 3.6, further1: 0.1 to 1.2: 0.4 to 3.1: 0.10 to 0.9: 0.4 to 3.1: 0.5 to 3.0, further 1: 0.16 to 1.0: 0.6 to 2.4: 0.14 to 0.75: 0.6 to 2.5: 0.6 to 2.4, further 1: 0.2 to 0.8: 0.8 to 2.1: 0.18 to 0.6: 0.8 to 2.0: 0.7 to 2.0, further 1: 0.2 to 0.7: 0.8 to 1.6: 0.2 to 0.5: 0.8 to 1.8: 0.8 to 1.4, further 1: 0.2 to 0.6: 1.0 to 1.2: 0.2 to 0.4: 0.9 to 1.7: 1.0 to 1.3, or 1.2: 0.05 to 1.6: 0.2 to 4.6: 0.05 to 1.2: 0.2 to 3.5: 0.25 to 3.6, further 1: 0.1 to 1.2: 0.4 to 3.1: 0.10 to 0.9: 0.4 to 3.1: 0.5 to 3.0, further 1: 0.16 to 1.0: 0.6 to 2.4: 0.14 to 0.75: 0.6 to 2.5: 0.6 to 2.4, further 1: 0.2 to 0.8: 0.8 to 2.1: 0.18 to 0.6: 0.8 to 2.0: 0.7 to 2.0, further 1: 0.2 to 0.7: 0.8 to 1.6: 0.2 to 0.5: 0.8 to 1.8: 0.8 to 1.4, and further 1: 0.2 to 0.6: 1.0 to 1.2: 0.2 to 0.4: 0.9 to 1.7: 1.0 to 1.3.

Specifically, the composition further comprises rutin, and a mass ratio of the isoquercitrin: the rutin is 1: 0.001 to 0.08, preferably 1: 0.005 to 0.06, preferably 1: 0.006 to 0.05, preferably 1: 0.007 to 0.04, preferably 1: 0.008 to 0.03, and preferably 1: 0.009 to 0.03, further, a content of the quercetin-3'-O-glucoside in the flavonoid constituent is 12.1% to 25%, and still further a content of the quercetin-3'-O-glucoside is 12.6% to 23% or 13% to 20%, and further, the composition comprises 0.08% to 2.5% of quercetin-3-O-robinobioside, preferably 0.1% to 1.5%, further 0.1% to 0.9%, and still further 0.1% to 0.5%.

More specifically, the composition comprises a total of more than 55% of quercetin, quercetin-3'-O-glucoside, myricetin, gossypetin-8-O-β-D-glucuronide, isoquercitrin, rutin and hyperoside, preferably more than 60%, preferably 65% to 85%, preferably 69% to 82%.

In a sixth aspect, the present invention provides a pharmaceutical composition, wherein the pharmaceutical composition comprises the above active fraction, the above Abelmoschus manihot extract or the above composition, and the pharmaceutical composition further comprises a pharmaceutically acceptable carrier.

Specifically, the pharmaceutically acceptable carrier comprises a solvent, an emulsifier, a disintegrant, a filler, a solubilizer, an antioxidant, a pH regulator, an osmotic pressure regulator, a bacteriostatic agent, a diluent, a lubricant, an adhesive and/or a film-forming agent, and the lubricant is free from magnesium stearate.

Specifically, the dosage form of the pharmaceutical composition comprises injection, tablet, suppository, ointment, gel, pill, granule, capsule or mixture, suspension and dispersible tablet.

In a seventh aspect, the present invention provides an application of the above active fraction, the above Abelmoschus manihot extract, the above composition or the above pharmaceutical composition in preparation of a drug for treating kidney disease, wherein the kidney disease is preferably diabetic kidney disease, or diabetic kidney disease or nephritis with renal fibrosis.

In an eighth aspect, the present invention provides an application of the above active fraction, the above Abelmoschus manihot extract, the above composition or the above pharmaceutical composition in preparation of a drug for treating eye disease, wherein the eye disease is preferably macular degeneration, visual fatigue or cataract, diabetic retinopathy, age-related macular degeneration, central exudative choroiretinitis, or macular degeneration caused by high myopia.

In a ninth aspect, the present invention provides an application of the above active fraction, the above Abelmoschus manihot extract, the above composition or the above pharmaceutical composition in preparation of a drug for treating lupus nephritis, contrast-induced kidney injury, pulmonary fibrosis or cardiac failure, or for lowering uric acid, or in preparation of an SGLT2 inhibitor.

In a tenth aspect, the present invention provides a preparation method of the above active fraction or the above Abelmoschus manihot extract, comprising the steps of: (1) extracting an Abelmoschi corolla or a medicinal part of an Abelmoschus manihot with ethanol to obtain an extract solution; (2) concentrating the extract solution, and then leaching the concentrated extract solution to obtain a leached solution; and (3) removing solvent from the leached solution, and then eluting the leached solution with macroporous resin to obtain a flavonoid active fraction or an extract of the Abelmoschi corolla, wherein a preferred extraction method is percolation.

Specifically, in the step (1), a dosage of the ethanol is 10 times to 25 times that of the Abelmoschi corolla or the medicinal part of the Abelmoschus manihot, and the ethanol is a 60% to 95% ethanol solution; in the step (2), an extractant used for leaching is n-butanol, petroleum ether or ethyl acetate, a method of leaching is continuous countercurrent leaching, a material-to-liquid ratio for the leaching is 0.8 to 4: 1; and in the step (3), the leaching has 1 to 5 stages, and a model of the macroporous resin is D101, HPD100 or AB-8.

Specifically, the step (2) further comprises the step of subjecting the extract solution to activated carbon adsorption, alcohol precipitation or acid precipitation before leaching.

Specifically, an elution process for the macroporous resin in the step (3) is as follows: a diameter-to-height ratio of the macroporous resin is 1: 4 to 1: 9, a concentration of a loading solution is 0.10 g to 0.30 g crude drug/mL, a volume of the loading solution is 4 BV to 12 BV, a flow rate for loading the solution is 1 BV/h to 4 BV/h. Impurities are removed with 4 BV to 8 BV of pure water and 1 BV to 5 BV of 3% to 15% ethanol at a flow rate of 0.5 BV/h to 4 BV/h, and the solution is eluted with 2 BV to 8 BV of 50% to 80% ethanol at a flow rate of 1 BV/h to 5 BV/h.

In an eleventh aspect, the present invention provides a preparation method of the above active fraction or the above Abelmoschus manihot extract, comprising the steps of: (1) extracting an Abelmoschi corolla or a medicinal part of an Abelmoschus manihot with ethanol to obtain an extract solution; (2) adding a clarifying agent into the extract solution, subjecting the solution to water bath treatment, and filtering the solution to remove a supernatant; and (3) eluting the supernatant with polyamide resin to obtain a flavonoid active fraction or an extract of the Abelmoschi corolla, wherein a preferred extraction method is percolation.

Specifically, in the step (1), a dosage of the ethanol is 10 times to 25 times that of the Abelmoschi corolla or the medicinal part of the Abelmoschus manihot, and the ethanol is a 60% to 95% ethanol solution; in the step (2), the water bath is carried out at a temperature of 50°C to 70°C and lasts for 30 minutes to 90 minutes; and in the step (3), a diameter-to-height ratio of the resin is 1: 4 to 1: 9, a concentration of a loading solution is 0.10 g to 0.30 g crude drug/mL, a volume of the loading solution is 4 BV to 12 BV, and the elution is carried out with 4 BV to 8 BV of pure water and 4 BV to 8 BV of 60% to 95% ethanol.

In a twelfth aspect, the present invention provides a preparation method of the above active fraction or the above Abelmoschus manihot extract, comprising the steps of: (1) extracting an Abelmoschi corolla or a medicinal part of an Abelmoschus manihot with ethanol to obtain an extract solution; (2) adjusting a pH value of the extract solution to be 2.0 to 3.0, refrigerating, and filtering the extract solution to obtain a precipitate, and adding water to dissolve the precipitate; (3) leaching the dissolution solution to obtain a leached solution; and (4) removing solvent from the leached solution, and then eluting the leached solution with polyamide resin to obtain a flavonoid active fraction or an extract of the Abelmoschi corolla, wherein a preferred extraction method is refluxing.

Specifically, in the step (1), a dosage of the ethanol used is 10 times to 25 times that of the Abelmoschi corolla or the medicinal part of the Abelmoschus manihot, and the ethanol is a 60% to 95% ethanol solution; in the step (2), a pH regulator is hydrochloric acid, sulfuric acid, acetic acid, phosphoric acid, citric acid, tartaric acid or maleic acid; in the step (3), an extractant used for leaching is n-butanol, petroleum ether or ethyl acetate, a method of leaching is continuous countercurrent leaching, a material-to-liquid ratio for the leaching is 0.8 to 4: 1; and the leaching has 1 to 5 stages; and in the step (4), a diameter-to-height ratio of the resin is 1: 4 to 1: 9, a concentration of a loading solution is 0.10 g to 0.60 g crude drug/mL, a volume of the loading solution is 4 BV to 12 BV, and the elution is carried out with 4 BV to 8 BV of pure water and 4 BV to 8 BV of 60% to 95% ethanol.

According to the Abelmoschi corolla flavonoid active fraction of the present invention, in addition to detecting the 7 constituents of hyperoside, rutin, isoquercitrin, gossypetin-8-O-β-D-glucuronide (hibifolin), myricetin, quercetin-3'-O-glucoside and quercetin, a content of quercetin-3-O-sophoricoside in a solid sample prepared by the embodiment of the present invention is converted in proportion by a peak area at the same time in the detection process, a content conversion calculation method is that: converted content=peak area of hyperoside control/concentration of hyperoside control * correction factor * peak area of quercetin-3-O-sophoricoside * constant volume/sample weight (after water conversion), and the content is not higher than 2.0%, preferably not higher than 0.9%, and preferably 0.1% to 0.7%. The total content of various flavonoid constituents detected is above 70%, further above 75%, and further between 80% and 90%.Beneficial effects:
(1) The Abelmoschi corolla flavonoid active fraction or extract of the present invention have the function of treating various nephritis, such as diabetic kidney disease, renal injury caused by a contrast agent or lupus nephritis, and also have the function of treating the eye disease, especially various diseases related to inhibiting a blood flow and inhibiting eye blood vessels, such as age-related macular degeneration, central exudative chorioretinitis, macular degeneration caused by high myopia, and the like, and in particular, the flavonoid active fraction or extract of the Abelmoschi corolla also have the function of treating pulmonary fibrosis.
(2) The preparation method of the present invention has the advantages of simple process, short production period, mild treatment conditions, low energy consumption and good separation effect of the 7 Abelmoschi corolla flavonoid constituents. The raw materials and reagents used are widely available, low in cost, and easy to realize industrial mass production.
(3) The Abelmoschi corolla flavonoid active fraction or extract or composition of the present invention have lower side effects, and provide a better therapeutic means for patients.

### BRIEF DESCRIPTION OF THE DRAWINGS

FIG. 1 is a graph showing the detection results of pathological changes in rats after repeated administration of different drugs for three months; Among them, A is a normal group, B is a sample group of Embodiment 9.2, C is an Huangkui Capsule group, and D is a group of Embodiment 2-A4;
FIG. 2 is a graph showing the pathological results of DKD group, HK group and HT group; and
FIG. 3 is a graph showing the detection results of SLC5A2 mRNA expression in DKD, HK and HT groups.

### DETAILED EMBODIMENT

The present invention is further described in detail hereinafter with reference to specific embodiments, and the following embodiments are not intended to limit the present invention, and are only used to illustrate the present invention. Unless otherwise specified, the experimental methods used in the following embodiments, for which specific conditions are not specified, usually follow the conventional conditions, and the materials and reagents used in the following embodiments are all commercially available unless otherwise specified.

Abelmoschi corollas in the following embodiments are dried corollas of Abelmoschus manihot, and unless otherwise specified, the Abelmoschi corollas are the same batch of Abelmoschi corollas.

Contents of 7 constituents in an Abelmoschi corolla flavonoid active fraction prepared in each embodiment are determined. The 7 constituents refer to: hyperoside, rutin, isoquercitrin, gossypetin-8-O-β-D-glucuronide, myricetin, quercetin-3'-O-glucoside and quercetin. In a preparation process of the active fraction sample, contents of various ingredients in a solution of the active fraction before drying are also tested, and the contents in the sample before and after drying change to some extent. A UPLC method reported in *"*Quantitative Method Using One Marker for Simultaneousassay of Seven Constutents in Flowers of Abelmoschus Manihot" ("Chinese Journal of Pharmaceutical Analysis", No. 12, 2013, 2082-2087) is used as a detection method to determine the contents of the ingredients.

A material-liquid ratio (i.e., extractant: extract solution) in leaching refers to a volume ratio.i.e., extractant: extract solution

### Embodiment 1: Preparation of Abelmoschi corolla flavonoid active fraction

100 g of Abelmoschi corollas were crushed into coarse powder, and subjected to percolation extraction with 15 times of 60% ethanol to obtain an extract solution of the Abelmoschi corolla. The extract solution was subjected to n-butanol removal, diluted with water, adsorbed by activated carbon, and then subjected to continuous countercurrent leaching with n-butanol, wherein a material-to-liquid ratio (i.e., extractant: extract solution) was 1.5: 1, a sample injection speed of the leaching was 40 mL/min, a rotating speed of a leacher for leaching was 40 Hz , and a leaching stage was 2. The leached solution was subjected to solvent recovery under a reduced pressure at 40°C, and then processed with D101 macroporous resin, wherein a process technology of the macroporous resin was as follows: a diameter-to-height ratio of the macroporous resin was 1: 6, a concentration of a loading solution (aqueous solution) was 0.15 g crude drug/mL, a volume of the loading solution was 7 BV, and a flow rate for loading the solution was 2 BV/h. Impurities were removed with 6 BV of pure water and 3 BV of 5% ethanol at a flow rate of 2 BV/h, and the solution was eluted with 4 BV of 60% ethanol at a flow rate of 2 BV/h to obtain an eluate., and then the eluate was subjected to ethanol recovery under a reduced pressure at 50°C to obtain a flavonoid extract of the Abelmoschi corolla. Contents of 7 constituents were tested, and according to determination results of the contents, the contents were adjusted by an addition method, so that the contents of various constituents were as shown in the following table. There were 2.23 g of total solids of the Abelmoschi corolla flavonoid active fraction, and a total of 1769 mg of the 7 constituents, accounting for 79.32% of the total solids.

**Table 1 Content determination data of the sample**

| Constituent | Hyperos ide | Rutin | Isoquercitri n | Hibifolin | Myricetin | Quercetin-3'-O-glucoside | Quercetin |
|---|---|---|---|---|---|---|---|
| Content (mg) | 326.96 | 6.14 | 252.79 | 480.33 | 101.78 | 421.87 | 179.03 |
| Various constituents /isoquerceti n | 1.293 | 0.024 | 1.000 | 1.900 | 0.403 | 1.669 | 0.708 |
| Various constituents /hibifolin | 0.68 | 0.01 | 0.53 | 1 | 0.21 | 0.88 | 0.37 |
| Various constituents /total solids (%) | 14.66 | 0.28 | 11.34 | 21.54 | 4.56 | 18.92 | 8.03 |

### Embodiment 2: Preparation of Abelmoschi corolla flavonoid active fraction

### Embodiment 2-1

100 g of Abelmoschi corollas were crushed into coarse powder, and subjected to percolation extraction with 18 times of 70% ethanol to obtain an extract solution of the Abelmoschi corolla. The Abelmoschi corolla extract solution was subjected to ethanol removal, diluted with water, and subjected to continuous countercurrent leaching with ethyl acetate, wherein a material-liquid ratio (i.e., extractant: extract solution) in leaching was 2.5: 1, a sample injection speed of leaching was 80 mL/min, a rotating speed of a leacher for leaching was 40 Hz, and a leaching stage was 4. The leached solution was subjected to solvent recovery under a reduced pressure at 40°C, and then processed with D101 macroporous resin, wherein a processing technology of the macroporous resin was as follows: a diameter-height ratio of the macroporous resin was 1: 5, a concentration of a loading solution was 0.15 g crude drug/mL, a volume of the loading solution was 6 BV, and a flow rate for loading the sample was 2 BV/h. Impurities were removed with 6 BV of pure water and 3 BV of 5% ethanol at a flow rate of 2 BV/h, the solution was eluted with 4 BV of 60% ethanol at a flow rate of 1.5 BV/h to obtain an eluate, and then the eluate was subjected to ethanol recovery under a reduced pressure at 60°C to obtain a flavone extract of the Abelmoschi corolla. Contents of 7 constituents were tested, and according to determination results of the contents, the contents were adjusted by an addition method, so that the contents of various constituents were as shown in the following table, and 2.99 g of total solids of the Abelmoschi corolla flavonoid active fraction were prepared. A content of quercetin-3-O-robibioside was 0.28%.

**Table 2-1 Content determination data of the sample**

| Constituent | Hyperos ide | Rutin | Isoquercitri n | Hibifoli n | Myriceti n | Quercetin-3'-O-glucoside | Quercetin | Total of 7 constituent s |
|---|---|---|---|---|---|---|---|---|
| Content (mg) | 427.66 | 9.57 | 379.17 | 662.80 | 148.54 | 579.18 | 241.21 | 2448.13 |
| Various constituents/ isoquercetin | 1.128 | 0.025 | 1.000 | 1.748 | 0.392 | 1.527 | 0.636 | / |
| Various constituents/ hibifolin | 0.65 | 0.01 | 0.57 | 1 | 0.22 | 0.87 | 0.36 | 0.01 |
| Various constituents/ total solids (%) | 14.30 | 0.32 | 12.68 | 22.17 | 4.97 | 19.37 | 8.07 | 81.88 |

### Embodiment 2-A: Preparation of Abelmoschi corolla flavonoid active fraction

According to the method of Embodiment 2 above, the Abelmoschi corolla was enlarged by 10 times, and 2 batches of Abelmoschi corolla flavonoid active fractions were prepared separately, so as to obtain samples with Content determination data of the sample as shown in Table 2-2. A content of quercetin-3-O-robibioside was 0.20% to 0.50%.

**Table 2-2 Content determination data of the sample**

| Embodiment | Rutin | Hyperoside | Isoquercitrin | Hibifolin | Myricetin | Quercetin-3'-O-glucoside | Quercetin | Total of 7 constituent: |
|---|---|---|---|---|---|---|---|---|
| 2-A1 | 0.2 | 20.83 | 17.41 | 14.89 | 4.46 | 17.04 | 1.89 | 76.72 |
| 2-A2 | 0.15 | 18.19 | 13.6 | 17.33 | 4.67 | 16.12 | 3.94 | 73.99 |

In this table, 2-A1 comprises 0.24% of content that is converted in proportion by a peak area.

Proportions of various constituents were as shown in the following Table 2-3.

**Table 2-3 Proportion data of contents of various constituents in samples**

| Embodiment | Rutin | Hyperoside | Isoquercitrin | Hibifolin | Myricetin | Quercetin-3'-O-glucoside | Quercetin |
|---|---|---|---|---|---|---|---|
| 2-A1 | 0.01 | 1.20 | 1.00 | 0.86 | 0.26 | 0.98 | 0.11 |
| 2-A2 | 0.01 | 1.34 | 1.00 | 1.27 | 0.34 | 1.19 | 0.29 |
| 2-A1 | 0.01 | 1.40 | 1.17 | 1 | 0.30 | 1.14 | 0.13 |
| 2-A2 | 0.01 | 1.05 | 0.78 | 1 | 0.27 | 0.93 | 0.23 |

### Embodiment 2-A3

Hyperoside, isoquercitrin, hibifolin, myricetin, quercetin-3'-O-glucoside, quercetin and quercetin-3-O-sophoricoside were taken as raw materials, and mixed by a formula according to the ratio of Embodiment 2-A1 (replacing rutin with quercetin-3-O-sophoricoside, and adjusting the ratio to be 0.02), so as to prepare a total flavone composition.

### Embodiment 2-A4

Hyperoside, isoquercitrin, hibifolin, myricetin, quercetin-3'-O-glucoside, quercetin and quercetin-3-O-sophoricoside were taken as raw materials, and mixed by a formula according to the ratio of Embodiment 2-A1 (replacing rutin with quercetin-3-O-sophoricoside, and adjusting the ratio to be 0.05), so as to prepare a total flavone composition.

### Embodiment 2-B

In order to prepare the Abelmoschi corolla active fraction with quality conforming to a uniformity requirement, according to content results of various constituents in several batches of extracts of Embodiment 2-1, a content relationship between a medicinal material and the constituents of the extract was studied, and three batches of Abelmoschi corollas with different sources and greatly different constituents were mixed by software calculation. 2000 g of mixed Abelmoschi corollas were percolated and extracted with 18 times of 70% ethanol to obtain an extract solution of the Abelmoschi corolla. The Abelmoschi corolla extract solution was subjected to ethanol removal, diluted with water, and subjected to continuous countercurrent leaching with ethyl acetate, wherein a material-liquid ratio (i.e., extractant: extract solution) in leaching was 3: 1, a sample injection speed of leaching was 120 mL/min, a rotating speed of a leacher for leaching was 60 Hz, and a leaching stage was 4. The leached solution was subjected to solvent recovery under a reduced pressure at 50°C, added with water for dilution, and centrifuged, and a supernatant was taken and processed with D101 macroporous resin, wherein a processing technology of the macroporous resin was as follows: a diameter-height ratio of the macroporous resin was 1: 6, a concentration of a loading solution was 0.15 g crude drug/mL, a volume of the loading solution was 7 BV, and a flow rate for loading the sample was 2 BV/h. Impurities were removed with 6 BV of pure water and 3 BV of 5% ethanol at a flow rate of 2 BV/h, the solution was eluted with 4 BV of 60% ethanol at a flow rate of 2 BV/h to obtain an eluate, and the eluate was concentrated, dried under a reduced pressure at 60°C to obtain total solids, which contained 0.22% of rutin, 20.58% of hyperoside, 17.81% of isoquercitrin, 16.07% of hibifolin, 5.15% of myricetin, 17.37% of quercetin-3'-O-glucoside, 1.77% of quercetin. When the total solids contained quercetin-3-O-sophoricoside, the content was concerted by a peak area to be 0.43%. Mass proportions of various constituents were as shown in the following Table 2-4.

**Table 2-4 Proportion data of contents of various constituents in samples**

| Rutin | Hyperoside | Isoquercitrin | Hibifolin | Myricetin | Quercetin-3'-O-glucoside | Quercetin | Quercetin 3-O-robinobioside |
|---|---|---|---|---|---|---|---|
| 0.01 | 1.16 | 1.00 | 0.90 | 0.28 | 0.98 | 0.09 | 0.02 |
| 0.01 | 1.28 | 1.11 | 1.00 | 0.31 | 1.08 | 0.10 | 0.03 |

### Embodiment 3: Preparation of Abelmoschi corolla flavonoid active fraction

### Embodiment 3.1

100 g of Abelmoschi corollas were crushed into coarse powder, and subjected to percolation extraction with 18 times of 60% ethanol to obtain an extract solution of the Abelmoschi corolla. The Abelmoschi corolla extract solution was subjected to ethanol removal, diluted with water, and subjected to continuous countercurrent leaching with ethyl acetate, wherein a material-liquid ratio (i.e., extractant: extract solution) in leaching was 3: 1, a sample injection speed of leaching was 100 mL/min, a rotating speed of a leacher for leaching was 40 Hz, and a leaching stage was 5. The leached solution was subjected to solvent recovery under a reduced pressure at 40°C, and then processed with D101 macroporous resin, wherein a processing technology of the macroporous resin was as follows: a diameter-height ratio of the macroporous resin was 1: 8, a concentration of a loading solution was 0.15 g crude drug/mL, a volume of the loading solution was 8 BV, and a flow rate for loading the sample was 2 BV/h. Impurities were removed with 6 BV of pure water and 3 BV of 5% ethanol at a flow rate of 2 BV/h, the solution was eluted with 4 BV of 60% ethanol at a flow rate of 3 BV/h to obtain an eluate, and then the eluate was subjected to ethanol recovery under a reduced pressure at 60°C to obtain a flavone extract of the Abelmoschi corolla. Contents of 7 constituents were tested, and according to determination results of the contents, the contents were adjusted by an addition method, so that the contents of various constituents were as shown in the following table, and 3.18 g of total solids of the Abelmoschi corolla flavonoid active fraction were prepared. A content of quercetin-3-O-robibioside was 0.20% to 0.50%.

**Table 3 Content determination data of the sample**

| Constituent | Hyperos ide | Rutin | Isoquercitrin | Hibifoli n | Myricetin | Querceti n-3'-O-glucosid e | Quercetin | Total of 7 constituen ts |
|---|---|---|---|---|---|---|---|---|
| Content (mg) | 434.84 | 10.1 8 | 399.27 | 699.92 | 147.98 | 605.99 | 246.54 | 2544.72 |
| Various constituents/i soquercetin | 1.089 | 0.02 5 | 1.000 | 1.753 | 0.371 | 1.518 | 0.617 | / |
| Various constituents/ hibifolin | 0.62 | 0.01 | 0.57 | 1 | 0.21 | 0.87 | 0.35 | / |
| Various constituents/t otal solids (%) | 13.67 | 0.32 | 12.56 | 22.01 | 4.65 | 19.06 | 7.75 | 80.02 |

### Embodiment 3.2

According to the preparation method of Embodiment 3.1, 500 g of Abelmoschi corolla medicinal materials were subjected to repeated experiment to obtain 2 batches of content data as shown in the following Table 4. A content of quercetin-3-O-robibioside was 0.30% to 0.80%. A represented "various constituents/total solids (%)" and B represented "various constituents/isoquercitrin".

**Table 4**

| Batch | | Hyperoside | Rutin | Isoquercitrin | Hibifolin | Myricetin | Quercetin-3'-O-glucoside | Quercetin | Total of 7 constituents |
|---|---|---|---|---|---|---|---|---|---|
| 3.2-1 | A | 15.39 | 0.38 | 13.52 | 17.58 | 3.38 | 14.41 | 4.65 | 69.30 |
| | B | 1.14 | 0.03 | 1.00 | 1.30 | 0.25 | 1.07 | 0.34 | / |
| 3.2-2 | A | 14.78 | 0.55 | 15.35 | 13.12 | 3.27 | 13.46 | 5.04 | 65.57 |
| | B | 0.96 | 0.04 | 1.00 | 0.85 | 0.21 | 0.88 | 0.33 | / |

### Embodiment 4: Preparation of Abelmoschi corolla flavonoid active fraction

### Embodiment 4-1

100 g of Abelmoschi corollas were percolated and extracted with 18 times of 70% ethanol to obtain an extract solution of the Abelmoschi corolla. The Abelmoschi corolla extract solution was subjected to ethanol removal, diluted with water, and subjected to continuous countercurrent leaching with n-butanol, wherein a material-liquid ratio (i.e., extractant: extract solution) in leaching was 3: 1, a sample injection speed of leaching was 120 mL/min, a rotating speed of a leacher for leaching was 60Hz, and a leaching stage was 5. The leached solution was subjected to solvent recovery under a reduced pressure at 50°C, and then processed with D101 macroporous resin, wherein a processing technology of the macroporous resin was as follows: a diameter-height ratio of the macroporous resin was 1: 6, a concentration of a loading solution was 0.15 g crude drug/mL, a volume of the loading solution was 7 BV, and a flow rate for loading the sample was 2 BV/h. Impurities were removed with 6 BV of pure water and 3 BV of 5% ethanol at a flow rate of 2 BV/h, the solution was eluted with 4 BV of 60% ethanol at a flow rate of 2 BV/h to obtain an eluate, and then the eluate was subjected to ethanol recovery under a reduced pressure at 60°C to obtain an Abelmoschi corolla extract. Contents of 7 constituents were tested, and according to determination results of the contents, the contents were adjusted by an addition method, so that the contents of various constituents were as shown in the following table, and 2.05 g of total solids of the Abelmoschi corolla flavonoid active fraction were prepared.

**Table 5 Content determination data of the sample**

| Constituent | Hyperosid e | Rutin | Isoquercitrin | Hibifolin | Myricetin | Quercetin -3'-O-glucoside | Quercetin | Total of 7 constituent s |
|---|---|---|---|---|---|---|---|---|
| Content (mg) | 286.21 | 9.36 | 243.67 | 446.73 | 99.84 | 367.12 | 149.38 | 1602.31 |
| Various constituents /isoquerceti n | 1.175 | 0.03 8 | 1.000 | 1.833 | 0.410 | 1.507 | 0.613 | / |
| Various constituents /hibifolin | 0.64 | 0.02 | 0.55 | 1 | 0.22 | 0.82 | 0.33 | |
| Various constituents /total solids (%) | 13.96 | 0.46 | 11.89 | 21.79 | 4.87 | 17.91 | 7.29 | 78.16 |

### Embodiment 4-2

Abelmoschi corollas were subjected to percolation extraction with 18 times of 70% ethanol, and an extract solution was subjected to ethanol removal to be 0.5 g crude drug/mL to 1.0 g crude drug/mL, added with ZTC1+1-II clarifying agents with 4% of B component 5 and 2% of A component respectively, subjected to water bath at 60°C for 60 minutes, centrifuged, filtered, diluted, and processed with polyamide resin, wherein a processing technology of the resin was as follows: a diameter-height ratio of the resin was 1: 7, a concentration of a loading solution was 0.15 g crude drug/mL To 0.5 g crude drug/mL, a volume of the loading solution was 8 BV, the solution was eluted with 5 BV of pure water and 5 BV of 80% ethanol to obtain an eluate, and the eluate was concentrated, and dried under a reduced pressure at 60°C. Contents of 7 constituents were tested, and according to determination results of the contents, the contents were adjusted by an addition method, so that the contents of various constituents were as shown in the following table, and total solids of the Abelmoschi corolla flavonoid active fraction were obtained. A content of quercetin-3-O-sophoricoside was 0.39%.

| Constituent | Rutin | Hyperoside | Isoquercitrin | Hibifolin | Myricetin | Quercetin-3'-O-glucoside | Quercetin | Total flavonoid |
|---|---|---|---|---|---|---|---|---|
| Various constituents / total solid (%) | 0.31 | 13.64 | 13.27 | 17.12 | 3.86 | 15.26 | 6.95 | 70.42 |
| Various constituents / isoquercitrin | 0.02 | 1.03 | 1 | 1.29 | 0.29 | 1.15 | 0.52 | 0.02 |
| Constituents / hibifolin | 0.02 | 0.80 | 0.78 | 1 | 0.23 | 0.89 | 0.41 | 0.02 |

### Embodiment 5: Preparation of Abelmoschi corolla flavonoid active fraction

### Embodiment 5-1

100 g of Abelmoschi corollas were subjected to percolation extraction with 15 times of 70% ethanol to obtain an extract solution of the Abelmoschi corolla. The Abelmoschi corolla extract solution was subjected to ethanol removal, diluted with water, and subjected to continuous countercurrent leaching with n-butanol, wherein a material-liquid ratio (i.e., extractant: extract solution) in leaching was 2.5: 1, a sample injection speed of leaching was 80 mL/min, a rotating speed of a leacher for leaching was 50Hz, and a leaching stage was 4. The leached solution was subjected to solvent recovery under a reduced pressure at 50°C, and then processed with D101 macroporous resin, wherein a processing technology of the macroporous resin was as follows: a diameter-height ratio of the macroporous resin was 1: 6, a concentration of a loading solution was 0.2g crude drug/mL, a volume of the loading solution was 6 BV, and a flow rate for loading the sample was 2 BV/h. Impurities were removed with 1 BV of pure water and 4 BV of 5% ethanol at a flow rate of 2 BV/h, the solution was eluted with 3 BV of 60% ethanol at a flow rate of 2 BV/h to obtain an eluate, and the eluate was subjected to ethanol recovery under a reduced pressure at 60°C to obtain an Abelmoschi corolla extract. Contents of 7 constituents were tested, and according to determination results of the contents, the contents were adjusted by an addition method, so that the content of each constituent was as shown in the following table. 2.86 g of total solids of the Abelmoschi corolla flavonoid active fraction were prepared.

### Embodiment 5-2

Abelmoschi corollas were refluxed and extracted with 15 times of 70% ethanol, the extract solution was subjected to ethanol removal to be 0.5 g crude drug/mL to 1.0 g crude drug/mL, added with 10% hydrochloric acid solution to adjust a pH value to be 2.0 to 3.0, refrigerated, filtered and washed, and a precipitate was taken, added with water for dissolution, and subjected to continuous countercurrent extraction with ethyl acetate, wherein a material-liquid ratio (i.e., extractant: extract solution) in leaching was 3: 1, and a leaching stage was 4. The leached solution was subjected to solvent recovery under a reduced pressure at 50°C, added with water for dilution, and then processed with polyamide resin, wherein a processing technology of the resin was as follows: a diameter-height ratio of the resin was 1: 6, a concentration of a loading solution was 0.15 g crude drug/mL to 0.5 g crude drug/mL, a volume of the loading solution was 6 BV, the solution was eluted with 4 BV of pure water and 4 BV of 70% ethanol to obtain an eluate, and the eluate was concentrated, and dried under a reduced pressure at 60°C to obtain the products. A content of quercetin-3-O-robibioside was 0.47%.

| **Constituent** | **Rutin** | **Hyperoside** | **Isoquercitrin** | **Hibifolin** | **Myricetin** | **Quercetin-3'-O-glucoside** | **Quercetin** | **Total flavonoid** |
|---|---|---|---|---|---|---|---|---|
| Various constituents / total solid (%) | 0.2 | 20.29 | 16.44 | 13.07 | 5.2 | 18.18 | 2.91 | 76.28 |
| Various constituents / isoquercitrin | 0.01 | 1.23 | 1 | 0.80 | 0.32 | 1.11 | 0.18 | 0.01 |
| Various constituents / hibifolin | 0.02 | 1.55 | 1.26 | 1 | 0.40 | 1.39 | 0.22 | 0.02 |

### Embodiment 6: Preparation of Abelmoschi corolla flavonoid active fraction

100 g of Abelmoschi corollas were subjected to percolation extraction with 16 times of 60% ethanol to obtain an extract solution of the Abelmoschi corolla. The Abelmoschi corolla extract solution was subjected to ethanol removal, diluted with water, and subjected to continuous countercurrent leaching with ethyl acetate, wherein a material-liquid ratio (i.e., extractant: extract solution) in leaching was 2: 1, a sample injection speed of leaching was 100 mL/min, a rotating speed of a leacher for leaching was 40Hz, and a leaching stage was 4. The leached solution was subjected to solvent recovery under a reduced pressure at 50°C, and then processed with D101 macroporous resin, wherein a processing technology of the macroporous resin was as follows: a diameter-height ratio of the macroporous resin was 1: 6, a concentration of a loading solution was 0.3g crude drug/mL, a volume of the loading solution was 3 BV, and a flow rate for loading the sample was 2 BV/h. Impurities were removed with 3 BV of pure water and 3 BV of 5% ethanol at a flow rate of 2 BV/h, and the solution was eluted with 4 BV of 70% ethanol at a flow rate of 2 BV/h to obtain an eluate. Contents of 7 constituents were tested, and the contents were adjusted by adding 7 specific constituents, so that the contents of various constituents were as shown in the following table. Then, the eluate was subjected to ethanol recovery under a reduced pressure at 60°C to obtain an Abelmoschi corolla extract. Contents of 7 constituents were tested, and according to determination results of the contents, the contents were adjusted by an addition method, with an addition amount not greater than 10% of a total weight, so that the contents of various constituents were as shown in the following table, and 3.16 g of total solids of the Abelmoschi corolla flavonoid active fraction were prepared.

**Table 6 Content determination data of the sample**

| Constituent | Hyperosi de | Rutin | Isoquercitrin | Hibifoli n | Myricetin | Quercetin -3'-O-glucoside | Quercetin | Total of 7 constitu ents |
|---|---|---|---|---|---|---|---|---|
| Content (mg) | 396.27 | 8.32 | 203.99 | 651.22 | 116.93 | 423.88 | 153.92 | 1954.53 |
| Various constituents / isoquercitri n | 1.943 | 0.041 | 1.000 | 3.192 | 0.573 | 2.078 | 0.755 | / |
| Various constituents / hibifolin | 0.61 | 0.01 | 0.31 | 1 | 0.18 | 0.65 | 0.24 | / |
| Various constituents / total solid (%) | 12.54 | 0.26 | 6.46 | 20.61 | 3.70 | 13.41 | 4.87 | 61.85 |

### Embodiment 7: Preparation of Abelmoschi corolla flavonoid active fraction (Comparative Example)

### Embodiment 7-1

500 g of Abelmoschi corollas were crushed into coarse powder, and subjected to percolation extraction with 20 times of 70% ethanol to obtain an extract solution of the Abelmoschi corolla. The solution was subjected to solvent recovery under a reduced pressure, and then processed with D101 macroporous resin, wherein a processing technology of the macroporous resin was as follows: a diameter-height ratio of the macroporous resin was 1: 4, a concentration of a loading solution was 0.15 g crude drug/mL, a volume of the loading solution was 6 BV, and a flow rate for loading the sample was 2 BV/h. Impurities were removed with 7 BV of pure water and 4 BV of 5% ethanol at a flow rate of 2 BV/h, the solution was eluted with 4 BV of 60% ethanol at a flow rate of 1.5 BV/h to obtain an eluate, and then the eluate was subjected to ethanol recovery under a reduced pressure at 60°C to obtain 16.12 g of total solids of the Abelmoschi corollas. Contents of various constituents were as shown in the following table.

**Table 7 Content determination data of the sample**

| Constituent | Hyperos ide | Rutin | Isoquercitrin | Hibifolin | Myricetin | Querceti n-3'-O-glucosid e | Quercetin | Total of 7 constit uents |
|---|---|---|---|---|---|---|---|---|
| Content (mg) | 2908 | 58 | 2421 | 2670 | 310 | 2522 | 95 | 16120 |
| Various constituents / isoquercitri n | 1.20 | 0.02 | 1.00 | 1.10 | 0.13 | 1.04 | 0.04 | / |
| Various constituents / total solid (%) | 17.60 | 0.35 | 14.65 | 16.16 | 1.88 | 15.27 | 0.58 | 66.49 |

### Embodiment 7-2

100 g of Abelmoschi corollas were crushed into coarse powder, and subjected to percolation extraction with 18 times of 70% ethanol to obtain an extract solution of the Abelmoschi corolla. The solution was subjected to solvent recovery under a reduced pressure, and then processed with D101 macroporous resin, wherein a processing technology of the macroporous resin was as follows: a diameter-height ratio of the macroporous resin was 1: 5, a concentration of a loading solution was 0.15 g crude drug/mL, a volume of the loading solution was 6 BV, and a flow rate for loading the sample was 2 BV/h. Impurities were removed with 6 BV of pure water and 3 BV of 5% ethanol at a flow rate of 2 BV/h, the solution was eluted with 4 BV of 60% ethanol at a flow rate of 1.5 BV/h to obtain an eluate, and then the eluate was subjected to ethanol recovery under a reduced pressure at 60°C to obtain 3.56g of total solids of the Abelmoschi corollas.

**Table 7 Content determination data of the sample**

| Constituent | Hyperos ide | Rutin | Isoquercitrin | Hibifoli n | Myricetin | Quercetin -3'-O-glucoside | Quercetin | Total of 7 constit uents |
|---|---|---|---|---|---|---|---|---|
| Content (mg) | 582.48 | 13.2 2 | 454.17 | 495.16 | 73.96 | 437.40 | 33.12 | 2089.5 1 |
| Various constituents / isoquercitri n | 1.283 | 0.02 9 | 1.000 | 1.090 | 0.163 | 0.963 | 0.073 | / |
| Various constituents / total solid (%) | 16.36 | 0.37 | 12.76 | 13.91 | 2.08 | 12.29 | 0.93 | 58.69 |

### Embodiment 8: Preparation of Abelmoschi corolla flavonoid active fraction (Comparative Example)

100 g of Abelmoschi corollas were crushed into coarse powder, and subjected to percolation extraction with 18 times of 70% ethanol to obtain an extract solution of the Abelmoschi corolla. The Abelmoschi corolla extract solution was subjected to ethanol removal, diluted with water, and subjected to continuous countercurrent leaching with ethyl acetate, wherein a material-liquid ratio (i.e., extractant: extract solution) in leaching was 2.5: 1, a sample injection speed of leaching was 80 mL/min, a rotating speed of a leacher for leaching was 40 Hz, and a leaching stage was 4. The leached solution was subjected to solvent recovery under a reduced pressure at 40°C, and then processed with D101 macroporous resin, wherein a processing technology of the macroporous resin was as follows: a diameter-height ratio of the macroporous resin was 1: 5, a concentration of a loading solution was 0.15 g crude drug/mL, a volume of the loading solution was 6 BV, and a flow rate for loading the sample was 2 BV/h. Impurities were removed with 6 BV of pure water and 3 BV of 5% ethanol at a flow rate of 2 BV/h, and the solution was eluted with 4 BV of 60% ethanol at a flow rate of 1.5 BV/h to obtain an eluate. Contents were tested. Then, the eluate was added with rutin and hyperoside, so that the contents of various constituents were as shown in the following table, and then the eluate was subjected to ethanol recovery under a reduced pressure at 60°C to obtain 4.28 g of total solids of the Abelmoschi corollas.

**Table 8 Content determination data of the sample**

| Constituent | Hyperos ide | Rutin | Isoquercitrin | Hibifolin | Myricetin | Querceti n-3'-O-glucosid e | Quercetin | Total of 7 constitu ents |
|---|---|---|---|---|---|---|---|---|
| Content (mg) | 1742.42 | 66.53 | 382.26 | 668.04 | 144.57 | 583.08 | 243.28 | 3830.18 |
| Various constituents / isoquercitri n | 4.558 | 0.173 | 1.000 | 1.748 | 0.216 | 1.525 | 0.636 | / |
| Various constituents / total solid (%) | 40.71 | 1.55 | 8.93 | 15.61 | 3.38 | 13.62 | 5.68 | 89.49 |

### Embodiment 9

### Embodiment 9.1 Preparation of Abelmoschi corolla flavonoid active fraction

25 kg of Abelmoschi corollas were crushed into coarse powder, and subjected to percolation extraction with 18 times of 70% ethanol to obtain an extract solution of the Abelmoschi corolla. The Abelmoschi corolla extract solution was subjected to ethanol removal, diluted with water, and subjected to continuous countercurrent leaching with ethyl acetate, wherein a material-liquid ratio (i.e., extractant: extract solution) in leaching was 2.5: 1, and a leaching stage was 4. The leached solution was subjected to solvent recovery under a reduced pressure at 40°C, and then processed with D101 macroporous resin, wherein a processing technology of the macroporous resin was as follows: a diameter-height ratio of the macroporous resin was 1: 5, a concentration of a loading solution was 0.15 g crude drug/mL, a volume of the loading solution was 3 BV, and a flow rate for loading the sample was 1.5 BV/h. Impurities were removed with 6 BV of pure water and 3 BV of 10% ethanol at a flow rate of 2 BV/h, the solution was eluted with 4 BV of 60% ethanol at a flow rate of 1.5 BV/h to obtain an eluate, and then the eluate was subjected to ethanol recovery under a reduced pressure at 60°C to obtain total solids of the Abelmoschi corollas. 7 constituents were adjusted by adding 7 specific constituents (contents of added constituents were not greater than 20% of a total weight), so that the contents of various constituents were as shown in the following table, and total solids of the Abelmoschi corolla flavonoid active fraction were prepared. A content of quercetin-3-O-robibioside was 0.20% to 0.50%.

**Table 9-1 Content determination data of the sample**

| Constituent | Hyperos ide | Rutin | Isoquercitrin | Hibifoli n | Myricetin | Quercetin -3'-O-glucoside | Quercetin | Total of 7 constitu ents |
|---|---|---|---|---|---|---|---|---|
| Various constituents / total solid (%) | 13.80 | 0.14 | 13.63 | 13.84 | 4.33 | 15.63 | 7.52 | 69.00 |
| Various constituents / isoquercitrin | 1.00 | 0.011 | 1.00 | 1.00 | 0.333 | 1.167 | 0.556 | |

### Embodiment 9.2 Preparation of Abelmoschi corolla flavonoid active fraction

With reference to Embodiment 2-B, 30 kg of Abelmoschi corolla mixtures were crushed into coarse powder, and subjected to percolation extraction with 18 times of 70% ethanol to obtain an extract solution of the Abelmoschi corolla. The Abelmoschi corolla extract solution was subjected to ethanol removal, diluted with water, and subjected to continuous countercurrent leaching with ethyl acetate, wherein a material-liquid ratio (i.e., extractant: extract solution) in leaching was 2.5: 1, a sample injection speed of leaching was 80 mL/min, a rotating speed of a leacher for leaching was 40 Hz, and a leaching stage was 4. The leached solution was subjected to solvent recovery under a reduced pressure at 40°C, and then processed with D101 macroporous resin, wherein a processing technology of the macroporous resin was as follows: a diameter-height ratio of the macroporous resin was 1: 5, a concentration of a loading solution was 0.15 g crude drug/mL, a volume of the loading solution was 5 BV, and a flow rate for loading the sample was 1.5 BV/h. Impurities were removed with 3 BV of pure water and 4 BV of 5% ethanol at a flow rate of 2 BV/h, the solution was eluted with 5 BV of 60% ethanol at a flow rate of 1.5 BV/h to obtain an eluate, and then the eluate was subjected to ethanol recovery under a reduced pressure at 65°C to obtain total solids of the Abelmoschi corollas. The total solids were dried in vacuum and crushed, and contents (%) of 7 flavone constituents were detected. Results were as follows, wherein a content of quercetin-3-O-sophoricoside was 0.20% to 0.50%.

**Table 10 Content determination data of the sample**

| Content of the constituents | Hyperosi de | Rutin | Isoquercitrin | Hibifolin | Myricetin | Querceti n-3'-O-glucosid e | Querceti n | Total of 7 constit uents |
|---|---|---|---|---|---|---|---|---|
| Various constituents / total solid (%) | 19.80 | 0.15 | 16.83 | 15.06 | 4.45 | 17.05 | 3.70 | 77.16 |
| Various constituents / isoquercitrin | 1.178 | 0.009 | 1 | 0.911 | 0.267 | 1.022 | 0.222 | |

### Embodiment 11: Preparation of eye drops of Abelmoschi corolla flavonoid active fraction

Preparation process: the active fraction was prepared according to any one of the methods of Embodiments 1 to 7, added with injection water for dissolution under stirring to reach 100 L, adjusted to allow a pH value to be 7, filtered and packed, and sterilized to obtain the eye drops. The pH regulator used was sodium hydroxide or/and hydrochloric acid.

### Embodiment 12: Pharmacological experiment of diabetic kidney disease model

### Embodiment 12-1

Animal model: streptozotocin (STZ)+high-fat-fed diabetic kidney disease model, C57BL/6 mice, 20±10 g, SPF grade, male.

Administration method: intragastric administration.

Experimental modeling: after 3 days of adaptive feeding, the experimental mice were fed with high-fat and high-sugar feed (made by laboratory according to a formula of lard: sucrose: yolk: basic feed=18: 20: 3: 59) for 8 consecutive weeks, and then intraperitoneally injected with an STZ sodium citrate solution according to a dosage of 100 mg/kg for 7 consecutive days. On the 8th day, blood glucose levels of the mice were detected by tail vein, and a blood glucose level greater than 16.7 mmol/L was taken as a modeling standard of the diabetic kidney disease model. When the blood glucose was greater than 13.8 mmol/L, and there were urine protein and renal function abnormality, the modeling was successful.

Experimental groups: there were a total of 6 groups, comprising a normal group, a model group, a positive drug group (Dapagliflozin tablet, 45.5 mg/kg), a sample group prepared by the method of Embodiment 2-1 (62.4 mg/kg), a sample group prepared by the method of Embodiment 6 (62.4 mg/kg) and a sample group prepared by the method of Embodiment 8 (62.4 mg/kg), with 15 mice in each group. After 4 weeks of administration, urine protein and urine creatinine were detected, and a daily food intake and a mouse state were calculated. Detection results were as shown in the following Table 12-1.

**Table 12-1**

| Group | Urine protein/mg | Urine albumin-to-creatinine ratio | Diarrhea/number of mice | Abdominal distension/number of mice |
|---|---|---|---|---|
| Normal group | 19.37 ± 13.28 | 0.24 ± 0.08 | 0 | 0 |
| Model group | 58.36 ± 36.31* | 2.82 ± 1.12* | 0 | 0 |
| Positive drug group | 17.51 ± 12.34^{#} | 1.04 ± 1.09^{#} | 0 | 0 |
| Sample group of Embodiment 8 | 39.62 ± 18.67 | 1.81 ± 0.37^{#} | 3 | 2 |
| Sample group of Embodiment 2-1 | 20.15 ± 15.67^{#} | 1.04 ± 2.21^{#} | 1 | 0 |
| Sample group of Embodiment 6 | 29.74 ± 17.78 | 1.11 ± 0.98^{#} | 4 | 3 |

| | | | | |
|---|---|---|---|---|
| Note: * indicated that P < 0.05 when comparing with the normal control group; ^{#} indicated that P < 0.05 when comparing with the model control group; and & indicated that P < 0.05 when comparing with the group of Embodiment 2-1. | | | | |

All the groups of the embodiments could reduce the urine protein, wherein the sample group of Embodiment 2 could significantly reduce the urine protein and the urine albumin-to-creatinine ratio (ACR). Observation results of the mouse state showed that the mice with diarrhea and abdominal distension in the sample group of Embodiment 2-1 were significantly less than those in other groups.

### Embodiment 12-2

Animal model: db/db mice, 16 weeks old, and C57 BL/6 mice, 16 weeks old.

Administration mode: intragastric administration.

Experimental modeling: the db/db mice were raised for 17 weeks, and then urine protein was detected. When there were urine protein and renal function abnormality, the modeling was successful for later experiment.

Experimental groups: there were a normal group of the C57 BL/6 mice, a model group of the db/db mice, a positive drug group (Dapagliflozin tablet, 45.5 mg/kg), a sample group prepared by the method of Embodiment 3-1 (62.4 mg/kg), a sample group prepared by the method of Embodiment 9.1 (62.4 mg/kg), and a sample group prepared by the method of Embodiment 2-A3 (62.4 mg/kg). There were 6 groups in total, with 15 mice in each group. After 4 weeks of administration, urine protein, urine creatinine and a daily food intake were detected, and a mouse state was observed. Detection results were as shown in the following Table 12-2.

**Table 12-2**

| Group | Urine protein/mg | Urine albumin-to-creatinine ratio/ | Diarrhea/number of mice | Abdominal distension/number of mice |
|---|---|---|---|---|
| Normal group | 17.21 ± 10.07 | 0.29 ± 0.16 | 0 | 0 |
| Model group | 61.97 ± 32.17* | 2.68 ± 1.03* | 0 | 0 |
| Positive drug group | 15.09 ± 10.27^{#} | 1.17 ± 1.26^{#} | 0 | 0 |
| Sample group of Embodiment 3-1 | 17.28 ± 14.54 | 1.29 ± 1.21^{#} | 1 | 0 |
| Sample group of Embodiment 9-1 | 16.91 ± 13.32^{#} | 1.17 ± 1.91^{#} | 2 | 0 |
| Sample group of Embodiment 2-A3 | 19.38 ± 14.67^{#} | 1.31 ± 1.28^{#} | 1 | 1 |
| Sample group of Embodiment 7-1 | 36.42 ± 14.23 | 1.45 ± 1.29^{#} | 5 | 3 |

| | | | | |
|---|---|---|---|---|
| Note: * indicated that P < 0.05 when comparing with the normal control group; ^{#} indicated that P < 0.05 when comparing with the model control group; and & indicated that P < 0.05 when comparing with group of Embodiment 7-1. | | | | |

All the groups of the embodiments could reduce the urine protein, and all the groups could reduce the urine protein and the urine albumin-to-creatinine ratio (ACR). Observation results of the mouse state showed that the mice with diarrhea and abdominal distension in the sample groups of Embodiment 3-1 and Embodiment 9-1 were significantly less than those in other groups.

### Embodiment 13: Effect of flavonoid on ocular blood flow and choroidal neovascularization

Experimental animals: rats, 150 g to 180 g weight, SPF grade, half male and half female.

Administration mode: intragastric administration.

Experimental modeling: healthy male Brown-Norway rats were selected and anesthetized by intramuscular injection of ketamine (50 mg/kg), pupils of the rats were dilated with a mixed mydriatic of 0.5% tropicamide and neofolin, and 8 points were photocoagulated between great retinal vessels around an optic disc at a distance 2 to 4 disc diameters away from the optic disc under a 120D special front lens by a krypton yellow laser (Lumenis Company in the United States) (wavelength 568 nm, and parameters: spot diameter 100 µm, exposure time 0.1 second, and power 150 mW to 200 mW). When small bubbles produced after a Bruch's membrane was broken were found, the photocoagulation points were qualified, and the rats with retinal, choroidal or vitreous hemorrhage were excluded.

### Experimental groups:

Drug groups and administration dosages: there were a normal group, a model group, a positive control drug group (Verteporfin 1.35 mg/kg), a sample group of Embodiment 2-1 (69.9 mg/kg), a sample group of Embodiment 3.1 (87.36 mg/kg), a sample group of Embodiment 7 (87.36 mg/kg), a sample group of Embodiment 9.1 (87.36 mg/kg) and a sample group of Embodiment 9.2 (87.36 mg/kg, solids prepared according to Embodiment 9.2), with 15 rats in each group. After 4 weeks of administration, an inhibition rate of choroidal blood flow and an area of choroidal neovascularization of eyes were detected. Detection results were shown in the following Table 13.

**Table 13**

| Group | Ocular choroidal blood flow inhibition ratio | Area of choroidal angiogenesis/µm² |
|---|---|---|
| Normal group | 0 | 0 |
| Model group | 39.78 ± 10.45* | 34.67 ± 1.78* |
| Positive drug control group | 87.98 ± 11.23^{#} | 19.80 ± 1.23^{#} |
| Sample group of Embodiment 2-1 | 80.67 ± 13.78^{#} | 23.24 ± 1.33^{#} |
| Sample group of Embodiment 3 | 83.76 ± 14.68^{#} | 22.89 ± 1.34^{#} |
| Sample group of Embodiment 7-2 | 61.98 ± 16.21^{#} | 28.78 ± 1.45^{#} |
| Sample group of Embodiment 9.1 | 84.19 ± 15.92^{#} | 24.57 ± 1.39^{#} |
| Sample group of Embodiment 9.2 | 85.27 ± 11.34^{#} | 23.79 ± 1.41^{#} |

| | | |
|---|---|---|
| Note: * indicated that P < 0.01 when comparing with the normal control group, and ^{#} indicated that P < 0.05 when comparing with the model control group. | | |

### Conclusion:

All the samples of the embodiments could inhibit the inhibition rate of choroidal blood flow and the area of choroidal neovascularization, and the sample groups of Embodiment 2, Embodiment 3, Embodiment 9.1 and Embodiment 9.2 could significantly reduce choroidal neovascularization.

### Embodiment 14: Effect of flavonoid on contrast-induced kidney injury

Experimental animals: SD rats, 200 g to 250 g weight, SPF grade, half male and half female.

Administration mode: intragastric administration.

Experimental modeling: healthy male SD rats were selected, intramuscularly injected with furosemide (10 ml/kg) first, and then injected with a prostaglandin synthesis inhibitor (indomethacin, 10 mg/kg, dissolved in dimethyl sulfoxide, and diluted with 1.25% of NAHCO3 solution), a nitric oxide synthase inhibitor (N-nitro-L-arginine-methyl ester hydrochloride, L-NAME, 10 mg/kg, dissolved in 0.9% of NaCl solution) and 76% of meglumine diatrizoate by tail vein every 15 minutes.

A success criteria of modeling indicated that a diagnostic criteria of acute kidney injury recommended by 2012 KDIGOAKI Clinical Practice Guideline (the diagnosis could be made when one of the following conditions was met): ① serum creatinine was increased to be more than 26.5 umol/L (0.3 mg/dl) within 48 hours; ② the increase of serum creatinine exceeded a baseline by 50%, which was confirmed or presumed to occur within 7 days; and ③ a urine volume was decreased to be less than 0.5 ml/(kg*h), which lasted for more than 6 hours. In animal models, the increase of serum creatinine exceeding the baseline by 50% was often used as the diagnostic criterion of acute kidney injury, that was, the success criterion of modeling.

Drug groups and administration dosages: there were a normal group, a model group, a positive control drug group (Irbesartan 50 mg/kg/d), a sample group of Embodiment 2-1 (69.9 mg/kg), a sample group of Embodiment 3.1 (87.36 mg/kg), a sample group of Embodiment 7 (87.36 mg/kg), a sample group of Embodiment 9.1 (87.36 mg/kg) and a sample group of Embodiment 9.2 (87.36 mg/kg, the solids prepared according to Embodiment 9.2), with 15 rats in each group. After 4 weeks of administration, an inhibition rate of choroidal blood flow and an area of choroidal neovascularization of eyes were detected. Detection results were shown in the following Table 14.

**Table 14**

| Group | Serum creatinine | Serum urea nitrogen |
|---|---|---|
| Normal group | 10.12 ± 3.89 | 2.48 ± 0.89 |
| Model group | 35.21 ± 4.32* | 9.89 ± 0.56* |
| Positive drug control group | 16.67 ± 1.23^{#} | 3.01 ± 0.98^{#} |
| Sample group of Embodiment 2-A1 | 15.56 ± 4.39^{#} | 2.98 ± 0.34^{#} |
| Sample group of Embodiment 3.1 | 17.67 ± 2.78^{#} | 3.12 ± 0.67^{#} |
| Sample group of Embodiment 4-2 | 18.80 ± 4.67^{#} | 4.02 ± 0.91^{#} |
| Sample group of Embodiment 8 | 25.23 ± 9.89^{#} | 4.21 ± 1.21^{#} |
| Sample group of Embodiment 6 | 27.43 ± 8.79^{#} | 5.23 ± 0.78^{#} |

| | | |
|---|---|---|
| Note: * indicated that P < 0.01 when comparing with the normal group, and ^{#} indicated that P < 0.05 when comparing with the model group. | | |

### Conclusion:

The serum creatinine and the serum urea nitrogen of the rats with the contrast-induced kidney injury in the model group were significantly higher than those in various administration groups. The sample groups of Embodiment 9-1, Embodiment 2-A1, Embodiment 3.1, Embodiment 8 and Embodiment 6 could all reduce the serum creatinine and the serum urea nitrogen to varying degrees.

### Embodiment 15: Effect of flavonoid on lupus erythematosus nephritis

26 male MRL/lpr mice (lupus erythematosus nephritis mice) were selected as an observation group 1, with 18 g to 22 g weight and 13 weeks old, which were purchased from Nanjing Junke Bioengineering Co., Ltd., and 24 male C57BL/6 mice were selected as a normal group, with 18 g to 22 g weight and 13 weeks old, which were purchased from Wuhan Hualianke Biotechnology Co., Ltd.

Drug groups and administration dosages: there were a normal group, an MRL/lpr mouse model group, a positive control drug group (Dexamethasone 1 mg/kg), a sample group of Embodiment 2-B (69.9 mg/kg), a sample group of Embodiment 3.1 (87.36 mg/kg), a sample group of Embodiment 7 (87.36 mg/kg), a sample group of Embodiment 9.1 (87.36 mg/kg) and a sample group of Embodiment 8 (87.36 mg/kg, , solids prepared according to Embodiment 9.2), with 15 mice in each group. The mice were administrated for 4 weeks.

### Experimental indicators:

After administration, 24-hour urine was collected to detect urine protein. After the urine was collected, eyeballs of all the mice were taken for blood collection, and the collected blood was centrifuged to obtain serum for antinuclear antibody detection. Meanwhile, kidneys of the mice were taken out, quickly frozen in liquid nitrogen, and stored in a refrigerator at-80°C for kidney immune detection. Detection results were shown in the following Table 15.

**Table 15**

| Group | Urine protein | Logarithm of antinuclear antibody titers | IgG level of kidney | C3 level of kidney |
|---|---|---|---|---|
| Normal group | 13.89 ± 3.23 | 0.32 ± 0.45 | 0.78 ± 0.78 | 0.98 ± 0.22 |
| Model group | 37.23 ± 3.89* | 1.71 ± 0.21* | 2.21 ± 0.24* | 2.20 ± 0.34* |
| Positive drug control group | 11.67 ± 3.45^{#} | 0.81 ± 0.11^{#} | 1.32 ± 0.67^{#} | 1.21 ± 0.67^{#} |
| Sample group of Embodiment 2-B | 18.51 ± 2.34^{#} | 0.91 ± 0.23^{#} | 1.28 ± 0.56^{#} | 1.35 ± 0.44^{#} |
| Sample group of Embodiment 3.1 | 16.56 ± 2.45^{#} | 1.12 ± 0.34^{#} | 1.35 ± 0.67^{#} | 1.45 ± 0.23^{#} |
| Sample group of Embodiment 5-2 | 13.78 ± 4.21^{#} | 0.69 ± 0.67^{#} | 1.21 ± 0.32^{#} | 1.13 ± 0.88^{#} |
| Sample group of Embodiment 8 | 21.45 ± 3.56^{#} | 1.19 ± 0.98 | 1.89 ± 0.65^{#} | 1.67 ± 0.12^{#} |
| Sample group of Embodiment 7 | 22.89 ± 4.53^{#} | 1.20 ± 0.12 | 1.76 ± 0.56^{#} | 1.78 ± 0.35^{#} |

| | | | | |
|---|---|---|---|---|
| Note: * indicated that P < 0.01 when comparing with the normal group, and ^{#} indicated that P < 0.05 when comparing with the model group. | | | | |

### Conclusion:

The urine protein, the serum antinuclear antibody level, and IgG and C3 deposit rates in kidney tissues of the lupus erythematosus nephritis mice in the model group were significantly higher than those of various administration groups. However, the sample groups of Embodiment 9.1, Embodiment 2-B, Embodiment 3.1, Embodiment 8 and Embodiment 7 could all improve the urine protein, the serum antinuclear antibody level, the IgG and C3 deposition in the kidney tissues, and the sample groups of Embodiment 2-B, Embodiment 3.1 and Embodiment 5.2 had better effects.

### Embodiment 16: Toxicological effects of flavonoid

Experimental animals: rats, 150 g to 180 g weight, SPF grade, half male and half female.

Administration mode: intragastric administration.

There were a normal group, a Huangkui Capsule group (extract 8 g/kg), a sample group of Embodiment 2-A1 (5 g/kg) and a sample group of Embodiment 9.1 (8 g/kg), with 30 rats in each group, and the rats were administrated once a day for 12 consecutive weeks according to the administration dosage calculated according to the extract.

### Experimental results:

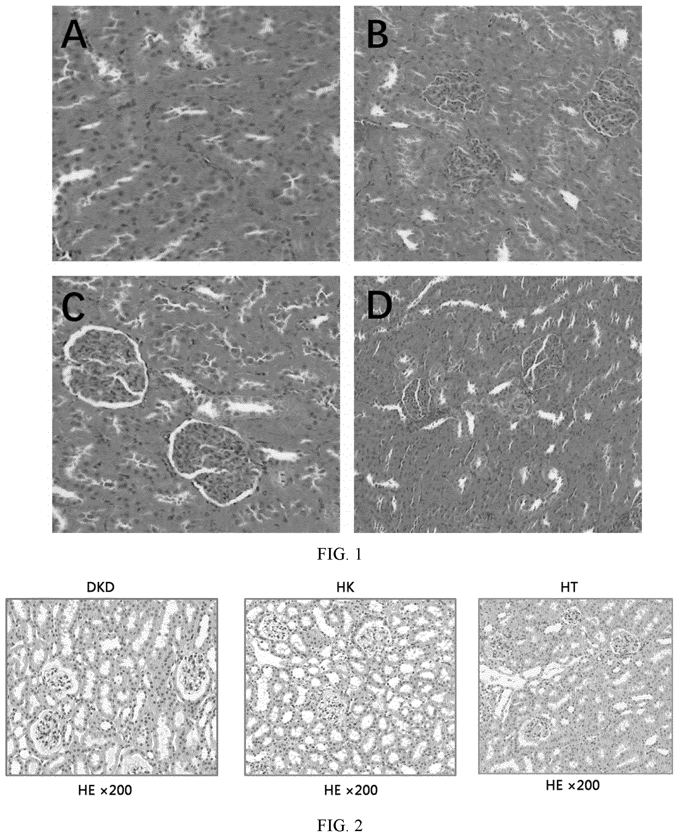

After administration, compared with the normal group, a kidney weight and viscera and brain coefficients of the Huangkui Capsule group were increased, and the pathology showed that the Huangkui Capsule group had moderate renal tubular hypertrophy. However, in the sample groups of Embodiment 9.2 and Embodiment 2-A1, the pathological examination showed no renal tubular hypertrophy. Detection results were as shown in the following Table 16 and figure 1 below.

**Table 16**

| Group | Kidney weight | Organ coefficient | |
|---|---|---|---|
| | | Liver | Spleen |
| Normal group | 3.390 ± 0.470 | 0.158 ± 0.013 | 2.477 ± 0.193 |
| Sample group of Embodiment 9.2 | 3.289 ± 0.321 | 0.159 ± 0.032 | 2.153 ± 0.156 |
| Huangkui Capsule group | 3.931 ± 0.433* | 0.234 ± 0.052* | 2.748 ± 0.190* |
| Sample group of Embodiment 2-A1 | 3.432 ± 0.345 | 0.187 ± 0.124 | 2.453 ± 0.134 |

| | | | |
|---|---|---|---|
| * indicated that P < 0.05 when comparing with the normal group. | | | |

### Embodiment 17: Effect of total flavonoid active fraction on idiopathic pulmonary fibrosis

Experimental animals: 60 Kunming mice, half male and half female, 18 g to 22 g weight, clean grade.

Animal groups: the mice were randomly divided into 6 groups, which comprise a normal group, a model group, a drug control group (5 mg/kg of Rosiglitazone), a sample group prepared by the method of Embodiment 2-A3 (70 mg/kg), a sample group prepared by the method of Embodiment 9.2 (70 mg/kg) and a Huangkui Capsule sample group (180 mg/kg based on the extract), with 10 mice in each group.

Experimental modeling: after 3 days of adaptive feeding, the experimental mice were anesthetized by intraperitoneal injection with 4% chloral hydrate (0.01 ml/g). Taking a supine fixed position, routine disinfection was carried out, a median cervical incision was made, and a trachea was exposed by passive separation. The model group, the control group and the treatment group were slowly injected with bleomycin (5 mg/kg) into a space between tracheal cartilaginous rings, and the normal control group was injected with the same volume of normal saline. The mice were immediately rotated upright for 3 minutes to 5 minutes after the injection, so that the liquid medicine was evenly distributed in both lungs, then skin of the mice was sutured and disinfected at a suture, and the mice after waking were sent to a clean grade observation room for feeding.

Administration mode: the mice were administrated on the second day after modeling, the administration group was administrated according to the above dosage once a day, and the normal group and the model group were administrated with the same volume of 10 ml/kg distilled water in the same way for 28 consecutive days.

Pathological section staining of lung tissue: after administration, a right lung was fixed, embedded, sectioned and stained with HE according to a routine pathological method. Degrees of pulmonary alveolitis and pulmonary fibrosis were divided into four grades.

The experimental results were shown in the following Table 17:

**Table 17**

| Group | Degree of pulmonary alveolitis | Degree of pulmonary fibrosis |
|---|---|---|
| Normal group | 0.43 ± 0.58 | 0.29 ± 0.38 |
| Model group | 1.69 ± 0.66* | 1.87 ± 0.69* |
| Drug control group | 1.03 ± 0.49^{#} | 1.15 ± 0.81^{#} |
| Sample group of Embodiment 2-A3 | 1.23 ± 0.55^{#} | 1.20 ± 0.67^{#} |
| Sample group of Embodiment 9.2 | 1.19 ± 0.68^{#} | 1.17 ± 0.90^{#} |
| Huangkui Capsule group | 1.48 ± 0.62^{#} | 1.69 ± 0.63^{#} |

| | | |
|---|---|---|
| Note: * indicated that P < 0.01 when comparing with the normal group, and ^{#} indicated that P < 0.05 when comparing with the model group. | | |

The experimental results showed that the degree of alveolitis and pulmonary fibrosis for the lung tissues of the mice in the model group was significantly aggravated, showing typical parenchymal pulmonary fibrosis lesions. After 28 days of administration, the lung coefficient of mice in each dose group was significantly reduced, and the degree of alveolitis and pulmonary fibrosis was alleviated remarkably. The total flavonoid active fraction could obviously reduce the degree of alveolitis and pulmonary fibrosis in mice with pulmonary fibrosis, and has a protective effect on the lungs of mice with pulmonary fibrosis, thereby alleviating the damage caused by fibrosis to the lung tissue.

### Embodiment 18: Study on action mechanism of SGLT2

Sodium-glucose con transporter 2 (SLC5A2, also known as SGLT2) was an encoding gene, which encoded a sodium-glucose cotransporter, and could reabsorb about 90% of glucose filtered by glomeruli. The SLC5A2 was only expressed in a brush border of an epithelial cell membrane of a renal proximal tubule, and was considered as a marker of the proximal tubule, with a main function of recovering filtered sodium and glucose. In a kidney tissue of a patient with diabetes and diabetic kidney disease, the inhibition of protein expression of the SLC5A2 could play a role in discharging sodium and water and lowering blood glucose, thus protecting the kidney tissue of the patient with diabetes and diabetic kidney disease.

Animal model: db/db mice, 18 weeks old.

Administration mode: intragastric administration.

Experimental groups: the db/db mice were divided into 3 groups when UACR > 200 mg/g, which were namely a diabetic kidney disease group (DKD), a Huangkui Capsule group (DKD+HK, 0.84 g/kg/d) and a total flavonoid group (DKD+HT, the sample of Embodiment 2-A2, 0.0755 g/kg/d), and the Huangkui Capsule group and the total flavonoid group were administrated with four weeks (28 days). Microalbumin (UACR) in urine of the db/db mice was detected by an enzyme-linked immunosorbant assay (ELISA) method, an mRNA expression level of the SLC5A2 was detected by a Reverse Transcription Polymerase Chain Reaction (RT-PCR) method, and a protein expression level of the SLC5A2 was detected by an ImmunoHistoChemistry (IHC) method.

### Experimental results:

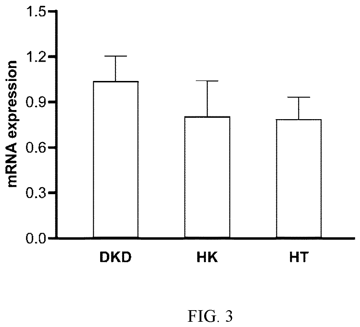

When the HK group (the Huangkui Capsule group) and the HT group (the total flavonoid group) were compared with the DKD group, the DKD (N=5), the HK (N=5) and the HT (N=6) had no significant difference in weight before grouping, and the DKD (N=5), the HK (N=5) and the HT (N=6) had no significant difference in weight in the first week and fourth week after administration. The DKD (N=5), the HK (N=5) and the HT (N=6) had no significant difference in blood glucose in the first week and fourth week after administration. The DKD (N=5), the HK (N=5) and the HT (N=6) had no significant difference in UACR value in the first week after administration. When the HT group was compared with the DKD group in the fourth week after administration, *P < 0.05, and the UACR value was significantly decreased, so that there was significant difference. The DKD (N=5), the HK (N=5) and the HT (N=6) had no significant difference in kidney mean value. According to kidney indicators of the DKD (N=5), the HK (N=5) and the HT (N=6), *P < 0.05, and the kidney indicators were significantly increased, so that there was significant difference. As shown in figure 2, according to the DKD group, some glomeruli were lobulated, a basement membrane was obviously thickened, glomerular capillaries were obviously compressed, and mesangial cells in a mesangial region proliferated and a mesangial matrix was obviously expanded; according to the HK group, the thickening of glomerular basement membrane was significantly improved, the glomerular capillaries were intact, and the proliferation of mesangial cells and mesangial matrix was significantly improved; and according to the HT group, the glomerular basement membrane was thinner obviously, there was no glomerular proliferation and glomerular capillary interstitial proliferation, and the proliferation of mesangial cells and mesangial matrix was not expanded. As shown in figure 3, IHC-P quantitative results showed that, compared with the DKD group (N=5), a protein expression level of SLC5A2 of the Huangkui HK group (N=5) and the HT group (N=6) was significantly reduced. Immunohistochemical results verified by RT-PCR results showed that, compared with the DKD group (N=5), an mRNA expression level of SLC5A2 of the Huangkui HK group (N=5) and the HT group (N=6) was significantly reduced. Compared with the DKD group (N=5), a ratio of an IHC-P quantitative expression quantity of SLC5A2 to a number of glomeruli of the Huangkui HK group (N=5) and the HT group (N=6) was significantly reduced, which was consistent with the IHC-P quantitative result.

Therefore, the Huangkui Capsule and the total flavonoid could effectively reduce the microalbuminuria of the db/db mice. The Huangkui Capsule and the total flavonoid could inhibit the protein expression of SLC5A2 on a cell membrane of a proximal convoluted tubule, reduce the protein expression of SLC5A2 in kidneys of the DKD mice, and effectively inhibit the protein activity of SLC5A2, thus effectively reducing the reabsorption of glucose in kidneys of the DKD mice.
The above embodiments merely express several embodiments of the present invention, and the descriptions thereof are more specific and detailed, but cannot be understood as a limitation to the scope of the invention patent. It should be noted that those of ordinary skills in the art may make a plurality of transformations and improvements without departing from the conception of the present invention, and these transformations and improvements should all fall within the scope of protection of the present invention. Therefore, the scope of protection of the invention patent should be subjected to the claims appended.

## Claims

1. A flavonoid active fraction of an Abelmoschi corolla, comprising the following flavonoid constituents in mass ratio:
a mass ratio of gossypetin-8-O-β-D-glucuronide : hyperoside : isoquercitrin : myricetin : quercetin-3'-O-glucoside being 10: 3.0 - 20: 4.0 : 18: 1.9 - 6.0: 6.0 -20.

2. The active fraction according to claim 1, wherein the mass ratio of the gossypetin-8-O-β-D-glucuronide: the hyperosidehyperoside: the isoquercitrin: the myricetin: the quercetin-3'-O-glucoside is 10: 4.0 to 17: 4.50 to 16: 1.9 to 5.5: 7.0 to 16, preferably 10: 6.0 to 15: 5.0 to 13: 2.0 to 5.0: 8.0 to 14, and further, the active fraction comprises a quercetin, wherein a mass ratio of the gossypetin-8-O-β-D-glucuronide: the quercetin is 10: 1.0 to 10.0, preferably 10: 1.0 to 6.0, and preferably 10: 1.5 to 5.0, still further, the active fraction comprises a rutin, wherein a mass ratio of the gossypetin-8-O-β-D-glucuronide: the rutin is 10: 0.05 to 0.6, preferably 10: 0.1 to 0.4, and further preferably 10: 0.1 to 0.3, and yet still further, the active fraction comprises a quercetin-3-O-robinobioside, wherein a mass ratio of the gossypetin-8-O-β-D-glucuronide: the quercetin-3-O-robinobioside is 10: 0.1 to 2.5, 10: 0.1 to 0.9, preferably 10: 0.15 to 0.5.

3. A flavonoid active fraction, comprising the following flavonoid constituents in mass ratio:
| | |
|---|---|
| isoquercitrin | 0.8 to 1.2 |
| quercetin | 0.05 to 1.6 |
| quercetin-3'-O-glucoside | 0.2 to 4.6 |
| myricetin | 0.05 to 1.2 |
| gossypetin-8-O-β-D-glucuronide | 0.2 to 3.5 |
| hyperoside | 0.25 to 3.6 |

4. The active fraction according to claim 3, wherein the active fraction comprises the following flavonoid constituents in mass ratio:
| | |
|---|---|
| isoquercitrin | 0.8 to 1.2 |
| quercetin | 0.1 to 1.2 |
| quercetin-3'-O-glucoside | 0.4 to 3.1 |
| | |
|---|---|
| myricetin | 0.10 to 0.9 |
| gossypetin-8-O-β-D-glucuronide | 0.4 to 3.1 |
| hyperoside | 0.5 to 3.0 |
preferably
| | |
|---|---|
| isoquercitrin | 0.8 to 1.2 |
| quercetin | 0.16 to 1.0 |
| quercetin-3'-O-glucoside | 0.6 to 2.4 |
| myricetin | 0.14 to 0.75 |
| gossypetin-8-O-β-D-glucuronide | 0.6 to 2.5 |
| hyperoside | 0.6 to 2.4 |
further preferably
| | |
|---|---|
| isoquercitrin | 0.8 to 1.2 |
| quercetin | 0.2 to 0.8 |
| quercetin-3'-O-glucoside | 0.8 to 2.1 |
| myricetin | 0.18 to 0.6 |
| gossypetin-8-O-β-D-glucuronide | 0.8 to 2.0 |
| hyperoside | 0.7 to 2.0 |
further preferably
| | |
|---|---|
| isoquercitrin | 0.8 to 1.2 |
| quercetin | 0.2 to 0.7 |
| quercetin-3'-O-glucoside | 0.8 to 1.6 |
| myricetin | 0.2 to 0.5 |
| gossypetin-8-O-β-D-glucuronide | 0.8 to 1.8 |
| hyperoside | 0.8 to 1.4 |
further preferably
| | |
|---|---|
| isoquercitrin | 0.8 to 1.2 |
| | |
|---|---|
| quercetin | 0.2 to 0.6 |
| quercetin-3'-O-glucoside | 1.0 to 1.2 |
| myricetin | 0.2 to 0.4 |
| gossypetin-8-O-β-D-glucuronide | 0.9 to 1.7 |
| hyperoside | 1.0 to 1.3 |
or
| | |
|---|---|
| isoquercitrin | 0.8 to 1.2 |
| quercetin | 0.09 to 1.6 |
| quercetin-3'-O-glucoside | 0.2 to 2.0 |
| myricetin | 0.05 to 1.2 |
| gossypetin-8-O-β-D-glucuronide | 0.5 to 3.0 |
| hyperoside | 0.25 to 3.6 |
or
| | |
|---|---|
| isoquercitrin | 0.8 to 1.2 |
| quercetin | 0.12 to 0.7 |
| quercetin-3'-O-glucoside | 0.8 to 1.6 |
| myricetin | 0.2 to 0.5 |
| gossypetin-8-O-β-D-glucuronide | 0.85 to 1.7 |
| hyperoside | 1.0 to 1.4. |

5. The active fraction according to any one of claims 3 to 5, wherein the isoquercitrin has a mass ratio value of 1.2 or 1.0.

6. The active fraction according to any one of claims 3 to 5, wherein the active fraction further comprises rutin, and a mass ratio of the isoquercitrin: the rutin is 1: 0.001 to 0.08, preferably 1: 0.005 to 0.06, preferably 1: 0.006 to 0.05, preferably 1: 0.007 to 0.04, and preferably 1: 0.009 to 0.04, or preferably 1: 0.008 to 0.03, preferably 1: 0.009 to 0.03, and further, the active fraction comprises quercetin-3-O-robinobioside, and a mass ratio of the isoquercitrin: the quercetin-3-O-robinobioside is 10: 0.1 to 2.5, 10: 0.1 to 0.9, and preferably 10: 0.15 to 0.5.

7. The active fraction according to any one of claims 1 to 5, wherein the active fraction comprises a total of more than 55% of quercetin, quercetin-3'-O-glucoside, myricetin, gossypetin-8-O-β-D-glucuronide, isoquercitrin and hyperoside, preferably more than 60%, preferably 65% to 85%, and preferably 69% to 82% further, the content of the quercetin-3'-O-glucoside is 12.1% to 25%, and still further, the content of the quercetin-3'-O-glucoside is 12.6% to 23% or 13% to 20%, 13% to 20% or 14% to 25%, or further, the content of the quercetin is higher than 0.7%, further higher than 1.0%, further 1.0% to 10%, further 1.5% to 5%, or still further, the gossypetin-8-O-β-D-glucuronide is 8.5% to 30%, further 12% to 23%.

8. The active fraction according to claim 7, wherein the active fraction comprises a total of more than 55% of quercetin, quercetin-3'-O-glucoside, myricetin, gossypetin-8-O-β-D-glucuronide, isoquercitrin, rutin and hyperoside, preferably more than 60%, preferably 65% to 85% or 66% to 84%, preferably 69% to 82% or 69% to 90%, and further the content of the quercetin-3'-O-glucoside is 12.1% to 25%, and still further the content of the quercetin-3'-O-glucoside is 12.6% to 23% or 13% to 20%.

9. A plant extract comprising flavonoid constituents, wherein the plant extract comprises the following constituents in mass ratio:
10% to 25% of hyperoside, 8% to 19% of isoquercitrin, 5% to 30% of gossypetin-8-O-β-D-glucuronide, and 12.1% to 25% of quercetin-3'-O-glucoside, further 12% to 23% of hyperoside, 10% to 17% of isoquercitrin, 8.5% to 25% of gossypetin-8-O-β-D-glucuronide, and 12.6% to 23% of quercetin-3'-O-glucoside, and further 13% to 22% of hyperoside, 11% to 17% of isoquercitrin, 12% to 21% of gossypetin-8-O-β-D-glucuronide, and 13% to 20% of quercetin-3'-O-glucoside.

10. The extract according to claim 9, wherein the extract comprises 2% to 10% of quercetin, preferably 2.5% to 9% of quercetin, preferably 3% to 8.5% of quercetin, and further 1.5-5% of quercetin, still further comprises 2% to 11% of myricetin, preferably 3% to 7% of myricetin, preferably 3% to 5% of myricetin, and further comprises 0.08% to 2.5% of quercetin-3-O-robinobioside, preferably 0.1% to 1.5%, further 0.1% to 0.9%, and still further 0.1% to 0.5%, and further, the plant is an Abelmoschi corolla or an Abelmoschus esculentus flower, preferably the Abelmoschi corolla.

11. An Abelmoschi corolla extract comprising flavonoid constituents, wherein the flavonoid constituent comprises the following constituents in mass ratio:
8% to 26% of hyperoside, 12.0% to 19.8% of isoquercitrin, 8.5% to 30% of gossypetin-8-O-β-D-glucuronide, 3.0% to 4.9% or 5.1% to 6.0% of myricetin, 14% to 25% of quercetin-3'-O-glucoside, and 1.6% to 4.9% of quercetin, and further 11% to 22% of hyperoside, 12.0% to 17% of isoquercitrin, 12% to 23% of gossypetin-8-O-β-D-glucuronide, 1.6% to 4.9% or 5.1% to 9.0% of myricetin, 13% to 22% of quercetin-3'-O-glucoside, and 1.4% to 8% or 1.4% to 7.8% of quercetin.

12. The Abelmoschi corolla extract according to claim 11, further comprising rutin at a mass content of 0.01% to 1.0%, further 0.05% to 0.8%, further 0.09% to 0.8%, and further 0.1% to 0.6%, and further comprises 0.08% to 2.5% of quercetin-3-O-robinobioside, preferably 0.1% to 1.5%, further 0.1% to 0.9%, and still further 0.1% to 0.5%.

13. The Abelmoschi corolla extract according to claim 12, wherein the flavonoid constituent in the Abelmoschi corolla extract has a mass content more than 55%, preferably more than 60%, preferably 65% to 85% or 66% to 84%, and preferably 69% to 82% or 69% to 90%.

14. A composition, comprising the following flavonoid constituents in mass ratio, wherein a mass ratio of isoquercitrin: quercetin: quercetin-3'-O-glucoside: myricetin: gossypetin-8-O-β-D-glucuronide: hyperoside is 1: 0.05 to 1.6: 0.2 to 4.6: 0.05 to 1.2: 0.2 to 3.5: 0.25 to 3.6, further1: 0.1 to 1.2: 0.4 to 3.1: 0.10 to 0.9: 0.4 to 3.1: 0.5 to 3.0, further 1: 0.16 to 1.0: 0.6 to 2.4: 0.14 to 0.75: 0.6 to 2.5: 0.6 to 2.4, further 1: 0.2 to 0.8: 0.8 to 2.1: 0.18 to 0.6: 0.8 to 2.0: 0.7 to 2.0, further 1: 0.2 to 0.7: 0.8 to 1.6: 0.2 to 0.5: 0.8 to 1.8: 0.8 to 1.4, further 1: 0.2 to 0.6: 1.0 to 1.2: 0.2 to 0.4: 0.9 to 1.7: 1.0 to 1.3, or 1.2: 0.05 to 1.6: 0.2 to 4.6: 0.05 to 1.2: 0.2 to 3.5: 0.25 to 3.6, further 1: 0.1 to 1.2: 0.4 to 3.1: 0.10 to 0.9: 0.4 to 3.1: 0.5 to 3.0, further 1: 0.16 to 1.0: 0.6 to 2.4: 0.14 to 0.75: 0.6 to 2.5: 0.6 to 2.4, further 1: 0.2 to 0.8: 0.8 to 2.1: 0.18 to 0.6: 0.8 to 2.0: 0.7 to 2.0, further 1: 0.2 to 0.7: 0.8 to 1.6: 0.2 to 0.5: 0.8 to 1.8: 0.8 to 1.4, and further 1: 0.2 to 0.6: 1.0 to 1.2: 0.2 to 0.4: 0.9 to 1.7: 1.0 to 1.3.

15. The composition according to claim 14, wherein the composition further comprises rutin, and a mass ratio of the isoquercitrin: the rutin is 1: 0.001 to 0.08, preferably 1: 0.005 to 0.06, preferably 1: 0.006 to 0.05, preferably 1: 0.007 to 0.04, preferably 1: 0.008 to 0.03, and preferably 1: 0.009 to 0.03, further, a content of the quercetin-3'-O-glucoside in the flavonoid constituent is 12.1% to 25%, and still further a content of the quercetin-3'-O-glucoside is 12.6% to 23% or 13% to 20%, and further, the composition comprises 0.08% to 2.5% of quercetin-3-O-robinobioside, preferably 0.1% to 1.5%, further 0.1% to 0.9%, and still further 0.1% to 0.5%.

16. The composition according to claim 15, wherein the composition comprises a total of more than 55% of quercetin, quercetin-3'-O-glucoside, myricetin, gossypetin-8-O-β-D-glucuronide, isoquercitrin, rutin and hyperoside, preferably more than 60%, preferably 65% to 85%, preferably 69% to 82%.

17. A pharmaceutical composition, wherein the pharmaceutical composition comprises the active fraction according to any one of claims 1 to 8, the Abelmoschus manihot extract according to any one of claims 9 to 13, or the composition according to any one of claims 14 to 16, and the pharmaceutical composition further comprises a pharmaceutically acceptable carrier.

18. The pharmaceutical composition according to claim 17, wherein the pharmaceutically acceptable carrier comprises a solvent, an emulsifier, a disintegrant, a filler, a solubilizer, an antioxidant, a pH regulator, an osmotic pressure regulator, a bacteriostatic agent, a diluent, a lubricant, an adhesive and/or a film-forming agent, and the lubricant is free from magnesium stearate.

19. The pharmaceutical composition according to claim 18, wherein the dosage form of the pharmaceutical composition comprises injection, tablet, suppository, ointment, gel, pill, granule, capsule, or mixture, suspension and dispersible tablet.

20. An application of the active fraction according to any one of claims 1 to 8, the Abelmoschus manihot extract according to any one of claims 9 to 13, the composition according to any one of claims 14 to 16, or the pharmaceutical composition according to any one of claims 17 to 19 in preparation of a drug for treating kidney disease, wherein the kidney disease is preferably diabetic kidney disease, or diabetic kidney disease or nephritis with renal fibrosis.

21. An application of the active fraction according to any one of claims 1 to 8, the Abelmoschus manihot extract according to any one of claims 9 to 13, the composition according to any one of claims 14 to 16, or the pharmaceutical composition according to any one of claims 17 to 19 in preparation of a drug for treating eye disease, wherein the eye disease is preferably macular degeneration, visual fatigue or cataract, diabetic retinopathy, age-related macular degeneration, central exudative choroiretinitis, or macular degeneration caused by high myopia.

22. An application of the active fraction according to any one of claims 1 to 8, the Abelmoschus manihot extract according to any one of claims 9 to 13, the composition according to any one of claims 14 to 16, or the pharmaceutical composition according to any one of claims 17 to 19 in preparation of a drug for treating lupus nephritis, contrast-induced kidney injury, pulmonary fibrosis or cardiac failure, or for lowering uric acid.

23. A preparation method of the active fraction of claims 1 to 8 or the Abelmoschus manihot extract of claims 9 to 13, comprising the steps of: (1) extracting an Abelmoschi corolla or a medicinal part of an Abelmoschus manihot with ethanol to obtain an extract solution; (2) concentrating the extract solution, and then leaching the concentrated extract solution to obtain a leached solution; and (3) removing solvent from the leached solution, and then eluting the leached solution with macroporous resin to obtain a flavonoid active fraction or an extract of the Abelmoschi corolla, wherein a preferred extraction method is percolation.

24. The preparation method according to claim 23, wherein in the step (1), a dosaget of the ethanol is 10 times to 25 times that of the Abelmoschi corolla or the medicinal part of the Abelmoschus manihot, and the ethanol is a 60% to 95% ethanol solution; an extractant used for leaching in the step (2) is n-butanol, petroleum ether or ethyl acetate, the leaching method is continuous countercurrent leaching, a material-to-liquid ratio for the leaching is 0.8 to 4: 1; and the leaching has 1 to 5 stages, and a model of the macroporous resin in the step (3) is D101, HPD100 or AB-8.

25. The preparation method according to claim 24, wherein the step (2) further comprises the step of subjecting the extract solution to activated carbon adsorption, alcohol precipitation or acid precipitation before leaching.

26. The preparation method according to claim 25, wherein an elution process for the macroporous resin is as follows: a diameter-to-height ratio of the macroporous resin is 1: 4 to 1: 9, a concentration of a loading solution is 0.10 g to 0.30 g crude drug/mL, a volume of the loading solution is 4 BV to 12 BV, a flow rate for loading the solution is 1 BV/h to 4 BV/h. Impurities are removed with 4 BV to 8 BV of pure water and 1 BV to 5 BV of 3% to 15% ethanol at a flow rate of 0.5 BV/h to 4 BV/h, and the solution is eluted with 2 BV to 8 BV of 50% to 80% ethanol at a flow rate of 1 BV/h to 5 BV/h.

27. A preparation method of the active fraction of claims 1 to 8 or the Abelmoschus manihot extract of claims 9 to 13, comprising the steps of: (1) extracting an Abelmoschi corolla or a medicinal part of an Abelmoschus manihot with ethanol to obtain an extract solution; (2) adding a clarifying agent into the extract solution, subjecting the solution to water bath treatment, and filtering the solution to remove a supernatant; and (3) eluting the supernatant with polyamide resin to obtain a flavonoid active fraction or an extract of the Abelmoschi corolla, wherein a preferred extraction method is percolation.

28. The preparation method according to claim 27, wherein in the step (1), a dosage of the ethanol is 10 times to 25 times that of the Abelmoschi corolla or the medicinal part of the Abelmoschus manihot, and the ethanol is a 60% to 95% ethanol solution; in the step (2), the water bath is carried out at a temperature of 50°C to 70°C and lasts for 30 minutes to 90 minutes; and in the step (3), a diameter-to-height ratio of the resin is 1: 4 to 1: 9, a concentration of a loading solution is 0.10 g to 0.30 g crude drug/mL, a volume of the loading solution is 4 BV to 12 BV, and the elution is carried out with 4 BV to 8 BV of pure water and 4 BV to 8 BV of 60% to 95% ethanol.

29. A preparation method of the active fraction of claims 1 to 8 or the Abelmoschus manihot extract of claims 9 to 13, comprising the steps of: (1) extracting an Abelmoschi corolla or a medicinal part of an Abelmoschus manihot with ethanol to obtain an extract solution; (2) adjusting a pH value of the extract solution to be 2.0 to 3.0, refrigerating, and filtering the extract solution to obtain a precipitate, and adding water to dissolve the precipitate; (3) leaching the dissolution solution to obtain a leached solution; and (4) removing solvent from the leached solution, and then eluting the leached solution with polyamide resin to obtain a flavonoid active fraction or an extract of the Abelmoschi corolla, wherein a preferred extraction method is refluxing.

30. The preparation method according to claim 29, wherein in the step (1), a dosage of the ethanol is 10 times to 25 times that of the Abelmoschi corolla or the medicinal part of the Abelmoschus manihot, and the ethanol is a 60% to 95% ethanol solution; in the step (2), a pH regulator is hydrochloric acid, sulfuric acid, acetic acid, phosphoric acid, citric acid, tartaric acid or maleic acid; in the step (3), an extractant used for leaching is n-butanol, petroleum ether or ethyl acetate, a method of leaching is continuous countercurrent leaching, a material-to-liquid ratio for the leaching is 0.8 to 4: 1; and the leaching has 1 to 5 stages; and in the step (4), a diameter-to-height ratio of the resin is 1: 4 to 1: 9, a concentration of a loading solution is 0.10 g to 0.60 g crude drug/mL, a volume of the loading solution is 4 BV to 12 BV, and the elution is carried out with 4 BV to 8 BV of pure water and 4 BV to 8 BV of 60% to 95% ethanol.
